(19) Europäisches Patentamt
European Patent Office
Office européen des brevets

(11) EP 0 784 459 B1

(12) EUROPEAN PATENT SPECIFICATION

(45) Date of publication and mention
of the grant of the patent:
**18.02.2004 Bulletin 2004/08**

(51) Int Cl.$^7$: **A61F 13/15**

(86) International application number:
**PCT/US1995/013273**

(21) Application number: **95937468.7**

(22) Date of filing: **05.10.1995**

(87) International publication number:
**WO 1996/010978 (18.04.1996 Gazette 1996/17)**

(54) **ABSORBENT SANITARY ARTICLE**

ABSORBIERENDES HYGIENEPRODUKT

ARTICLE HYGIENIQUE ABSORBANT

(84) Designated Contracting States:
**AT BE CH DE DK ES FR GB GR IE IT LI LU NL PT SE**

(30) Priority: **07.10.1994 IT TO940797**
**07.10.1994 IT TO940800**
**07.10.1994 IT TO940801**

(43) Date of publication of application:
**23.07.1997 Bulletin 1997/30**

(73) Proprietor: **THE PROCTER & GAMBLE COMPANY**
**Cincinnati, Ohio 45202 (US)**

(72) Inventors:
• **PALUMBO, Gianfranco**
**D-41348 Bad Homburg (DE)**

• **CARLUCCI, Giovanni**
**I-66100 Chieti (IT)**
• **MARINELLI, Luigi**
**I-65129 Pescara (IT)**
• **DI CINTIO, Achille**
**I-65126 Pescara (IT)**

(74) Representative: **Veronese, Pancrazio et al**
**Procter & Gamble Italia S.p.A.**
**Italian Research Center**
**Via Aterno 92/94**
**66020 Sambuceto di San Giovanni Teatino (Chieti) (IT)**

(56) References cited:
EP-A- 0 465 130       US-A- 1 643 926
US-A- 4 166 464       US-A- 5 201 727
US-A- 5 264 281       US-A- 5 429 630

**Description**

This invention relates to an absorbent sanitary article. It is particularly concerned with pantiliners, and will be so described, though it has application to other sanitary articles, for example sanitary napkins, incontinence products, and so forth.

Pantiliners on the market tend to be less than ideal in terms of the comfort with which they are worn. One reason for this is that they tend to bunch as a result of the movements of the user's body. Not only does this result in the user experiencing discomfort, but it also means that the pantiliner absorbs less fluid than it would otherwise absorb, as a result of the pantiliner being in inadequate contact with the user.

A non elastic sanitary article is for example disclosed in US 5 201 727, an elastic stitchbonded fabric is disclosed in EP-A-0 465 130.

It is an object of the present invention to provide a pantiliner which overcomes, or at least reduces, the above problems, and which is suitable for use in absorbing light fluid flows which may occur between menstrual periods. Such fluid flows include both vaginal discharge and urine. The construction of the pantiliner is to be such that it is able to move with the panty in which it is held, acting, so to speak, as an extension of the panty itself.

According to the present invention there is provided a multi-layer, fluid absorbent, sanitary article for use with a panty garment, the article having means of attachment to the interior of the garment in the crotch area thereof, the article being flexible, and elastic in at least one direction, the elasticity being such that in said at least one direction, the loading force required to extend a sample of material 1 inch (2.54cm) in width to 125% of its original length is not more than 400g, and the residual stretch (pad set) after the sample has been stretched three times to 125% of its original length and relaxed after each stretch is not more than 15% of its original length.

The said loading force is preferably not more than 250g, more preferably not more than 150g, and still more preferably not more than 100g, and is preferably at least 60g. The pad set is preferably not more than 12%, and preferably not more than 8%.

Preferably, the liner has a flexibility, as measured according to ASTM Standard D 1388-64, of not more than 5000 mg.cm, preferably not more than 3000 mg.cm, though the flexibility value is preferably at least 500 mg.cm. The pantiliner preferably has a thickness under a pressure of $20g/cm^2$, of less than 2mm, and more preferably in the range of from 0.5 to 1.6 mm.

The pantiliner of the invention will normally be generally elongate in shape, preferably having the hourglass shape which has now become widely accepted for such articles. The pantiliner may have elasticity in only one direction, in which case that direction is preferably transverse to the length of the pantiliner, or it may have elasticity in both the transverse and longitudinal directions.

There have been proposals in the prior art for pantiliners and catamenial articles which have elastic properties, and in this connection attention is directed to WO93/01785, US Patent 4166464, US Patent 4389211, EP-A-160517 and EP-A-450541. None, however, discloses the combination of properties which is set out above and which renders the article of the present invention so particularly suitable for its intended purpose.

The pantiliner of the present invention is preferably intended to absorb, in use, up to about 2g of fluid. For this level of discharge it is normally constructed to have an absorbent capacity of at least about 4 to 5 ml. This over-capacity is provided to take into account the fact that the fluid absorbed may all remain in the central region of the pantiliner, with the outer regions thereof remaining completely or substantially dry. This is likely to occur given the low flow rates and flow volumes which are involved.

The pantiliner according to the invention is made as a multilayer structure, at least one of these layers being fluid-absorbent. Each layer is adhered to the layer or layers immediately adjacent thereto. It has been found that since the article does not have to handle large fluid volumes it is unnecessary for the fluid-absorbent layer or layers to be completely enclosed between a topsheet and a backsheet, a construction which is often conventionally adopted to prevent fluid leaking from the absorbent material through the edges thereof. In the case of the article of the present invention, fluid almost never reaches those edges, and it is therefore of no consequence if those edges are exposed to the outside.

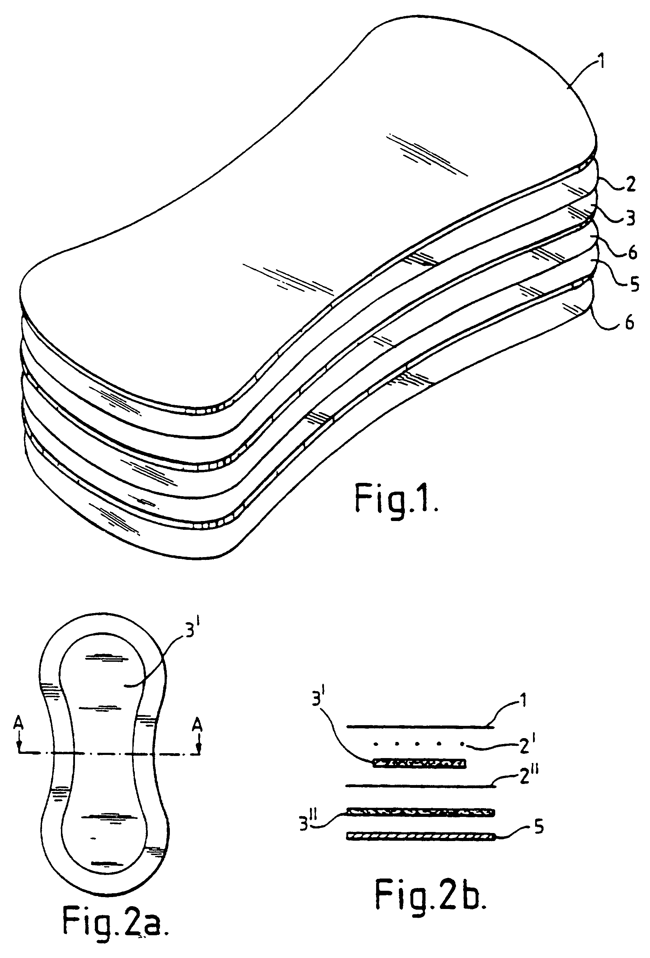

Accordingly, it is possible for the pantiliner of the present invention to consist of a plurality of layers of identical or substantially identical size and shape positioned in alignment with one another. This is shown in Figure 1 of the accompanying drawings.

In the accompanying drawings:

Figure 1 is an exploded isometric view of an article according to the present invention;
Figure 2a is a plan view of an alternative article according to invention;
Figure 2b is a section taken on line A-A in Figure 2a;
Figures 3 to 9 are graphs showing hysteresis cycles for several articles according to the invention;
Figure 10 illustrates diagrammatically a method of manufacturing a topsheet as just described;

Figure 11 is a stress-strain curve for an embodiment of such a topsheet;

Figure 12 is a hysteresis diagram for the embodiment of a topsheet to which Figure 11 relates;

Figure 13 is a hysteresis diagram for the elastic film used in that embodiment;

Figure 14 is a photomicrograph showing a topsheet material from below;

Figure 15 is a photomicrograph, on a larger scale, showing a transverse section through the material of Figure 14; and

Figure 16 is a perspective, diagrammatic view showing substantially what appears in Figure 15; and

Figures 17 to 27 show various patterns which can be used for applying panty fastening adhesive to sanitary articles.

Referring now more particularly to the exploded isometric view of Figure 1, this shows an article having a topsheet layer 1, adhesive 2, an absorbent core 3, adhesive 4, a backsheet 5, and adhesive 6. The nature and purpose of the various layers is discussed further below. The use of such a structure means that it is possible to produce a plurality of pantiliners by cutting them from overlaid sheets of the materials required, without the alignment problems which exist where a topsheet and a backsheet have to be arranged to form an envelope completely enclosing an absorbent core.

In order to provide the pantiliner with the necessary elastic properties, it is necessary in the case of a multilayer article for at least one of the layers to be elastic, though all the layers must be stretchable. It is found that the article as a whole has the necessary elastic properties even if some, but not all, of the layers are stretchable but not elastic.

In a further aspect thereof, the invention provides an elastic, fluid-absorbent liner for use with a panty garment, the liner having means of attachment to the interior of the garment in the crotch area thereof, the liner comprising a plurality of layers joined in face-to-face relationship with one another by means of at least one adhesive connection, the said adhesive connection or one of said adhesive connections, being provided by an elastic adhesive, whereby to provide, at least in part, the elasticity of the liner.

In another aspect, the invention provides a fluid-absorbent liner for.use with a panty garment, which comprises a layer of absorbent material exposed to the exterior on one face thereof, and joined in face-to-face relationship on its opposite face, by means of an adhesive connection, with a backsheet of water-impermeable material.

In yet another aspect, the invention provides an elastic, fluid-absorbent sanitary article for use with a panty garment, the article having means of attachment to the interior of the garment in the crotch area thereof, the article comprising a plurality of layers joined in face-to-face relationship with one another by means of at least one adhesive connection, the said adhesive connection or one of said adhesive connections, being provided by an elastic adhesive, whereby to provide, at least in part, the elasticity of the article.

In still another aspect the invention provides a fluid-absorbent sanitary article for use with a panty garment, which comprises a layer of absorbent material exposed to the exterior on one face thereof, and joined in face-to-face relationship on its opposite face, by means of an adhesive connection, with a backsheet of water-impermeable material.

The invention also provides a fluid-absorbing sanitary article, wherein the layers thereof, and the connections therebetween, are such as to render it permeable to water vapour and other gases.

Additionally, the invention provides a fluid-absorbing sanitary article for use with a cotton panty garment, the article having means for removably attaching it to the interior of the panty garment in the crotch area thereof, wherein the stress-strain characteristics of the said article correspond to a substantial extent to those of the said crotch area.

The following Table 1 sets out the function of the layers in a multilayer construction which consists successively, as considered from the body side of the user outwards, of six layers.

TABLE 1

| 1. | Topsheet | Dry feeling/skin friendly |
|---|---|---|
| 2. | Topsheet-to core adhesive | Material adhesion |
| 3. | Absorbent core | Absorbency |
| 4. | Core-to-backsheet adhesive | Material adhesion/optionally air & vapour permeability |
| 5. | Backsheet | Liquid impermeability /optionally air & vapour permeability |
| 6. | Backsheet-to-panty adhesive | Intimate panty bonding |

A description will now be given of examples of materials which can be used for the above layers. It should be noted at this point, however, that constructions are possible which use fewer layers, or more layers, than those just recited, and the materials mentioned below may be suitable for the layers of such constructions.

TOPSHEET

For a topsheet which is stretchable but not elastic, one suitable material is a perforated or apertured film which has been ring-rolled to provide it with a degree of extensibility. One suitable film is described in US Patent 4463045, and suitable processes for ring-rolling such a film are described in US Patents 4107364, 4834741, 5143679, 5156793 and 5167897. Ring-rolling has the effect of producing corrugations in the topsheet, and the ring-rolled topsheet has an axis of stretching which is perpendicular to the direction of the fold lines. The fold lines can all run in a single direction (i.e. parallel to one another), or in more than one direction (for example in two mutually parallel directions), and the sheet correspondingly has one or more axes along which it can be stretched.

Another possible material which can be ring-rolled to produce a stretchable topsheet is that described in EP-A-207904.

One possible material which can be used for a topsheet which is to be elastic, rather than just stretchable, will now be described.

By way of background, mention should be made of EP-A-207904, in which there is described a covering sheet for covering an absorbent body of an absorbent sanitary article, the said structure having perforations which extend therethrough and comprising an upper layer intended to face outwardly of the absorbent body and comprising a non-woven fibrous material, an intermediate layer comprising a film, and a lower layer intended to face inwardly towards the absorbent body and comprising a non-woven fibrous material. It has been found that an elastic topsheet can be made following a modified form of the teachings of EP-A-207904, replacing the intermediate layer used therein with an elastic film. This is surprising, since the process for joining the three layers together would have been expected, prima facie, to result in the elastic film no longer being able to exhibit its elastic properties, as a result of its connection on both sides to the fibrous layers. However, it has been found the process described in EP-A-207904 can be modified so that it does not in fact have this effect. The modification has the effect that the upper and lower layers are connected to the intermediate layer substantially only around the perimeters of the perforations, and this permits the elastic film to continue to exhibit its elasticity in at least one direction.

Thus, there can be provided, and a further aspect of the invention does so provide, a covering structure for covering an absorbent body of an absorbent sanitary article, the said structure having perforations which extend therethrough and being elastic in at least one direction, the structure comprising:

(a) an upper layer intended to face outwardly of the absorbent body and comprising a non-woven fibrous material;
(b) an intermediate layer comprising an elastic film; and
(c) a lower layer intended to face inwardly towards the absorbent body and comprising a non-woven fibrous material; the upper and lower layers being connected to the intermediate layer substantially only around the perimeters of the perforations.

The upper and lower layers may be formed of non-woven webs in which the individual fibres are aligned with one another to a substantial extent. Where that is so, the topsheet will be elastic in a direction transverse to the direction of alignment of the fibres, but substantially inelastic in a direction parallel to the direction of alignment. It follows from this that one cannot have the upper and lower layers with directions of alignment which are perpendicular to one another, since then the resulting topsheet would be substantially inelastic. On the contrary, one would normally have the directions of alignment the same in the upper and lower layers. If the fibres in either or both of the upper and lower layers are randomly oriented there will be some elasticity in all directions, but much less than the elasticity obtained when the fibres are oriented. It will be appreciated that there are intermediate possibilities between the two extremes of complete orientation and complete randomness, and that these will give intermediate amounts of elasticity and greater or lesser differences between the amount of elasticity in different directions.

The materials used for the upper and lower layers can be the same as those described in EP-A-207904, though they need not be. Thus, for example, both layers may be made of carded fibres, and these may be, for example, polypropylene fibres or two-component fibres sold under the name CHISSO ES by the Japanese company Chisso. The upper and lower layers may be hydrophobic, hydrophilic, or have at least one region which is hydrophobic and at least one region which is hydrophilic.

The material used for the elastic film is preferably based on a thermoplastic elastomer, and suitable materials include styrenic block copolymers (such as styrene-butadiene-styrene, styrene-isoprene-styrene, styrene-ethylene butylene-styrene and styrene-ethylene propylene-styrene copolymers), elastomeric polyurethanes, polyester and polyether elastomers and copolymers thereof, polyester-amides, poly-ether-ester amides and ionomers (polymeric materials in which chains are linked by ionic interactions). Other suitable materials include blends of or a polyolefin material (such as polypropylene, polyethylene, and copolymers thereof, in particular polyethylene vinyl acetate) with a rubber such as EPR or SBR rubber.

The film preferably has a thickness of from 8 to 100 $\mu$m, more preferably not more than 70 $\mu$m, and still more preferably from 30 $\mu$m to 50 $\mu$m. However, a still thinner film, having a thickness of, say 15-30 $\mu$m may be desirable. One particular film which can be used is that sold by the Exxon Chemical Corporation as EXX 500. This is available in a thickness of 50 $\mu$m. These values refer to the true thickness of the film. If an embossed film in used it may have a much greater apparent thickness, say 500 μm, including the embossings.

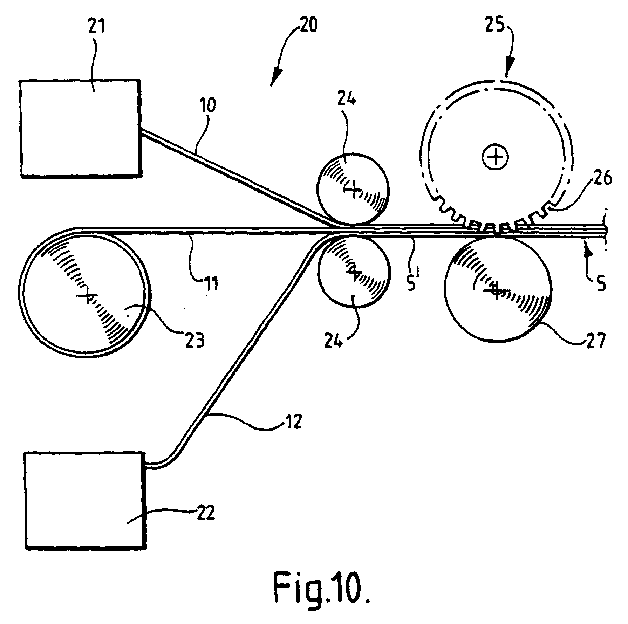

Further details will now be given referring to Figures 10 to 16 of the accompanying drawings.

Referring in more detail to Figure 10, this shows schematically a device generally indicated 120, which comprises two cards 121, 122 each of which continuously supplies a web of polypropylene or other suitable fibres. The web supplied by the card 121 forms an upper layer 110 and the web supplied by the card 122 forms the lower layer 112.

An elastic film intended to constitute an intermediate layer 111, is unwound continuously from a reel, generally indicated 23.

The webs 110 and 112 and the film 111 are fed towards two guide rollers 124 in a disposition such that the film 111 is interposed between the webs 110 and 112. The resulting assembly 105' is then directed to a perforating and bonding station, generally indicated 125, constituted by two counter-rotating superposed rollers 126, 127 with parallel axes. The lower roller 127, which acts as a rotary support for the assembly 105', has a generally smooth surface. The upper roller 126, however, has teeth or projections arranged in an array corresponding to that of the perforations which it is wished to make in the eventual product 105. At least the roller 126 is heated to a temperature sufficient to cause partial melting of those fibres of the web 110 into which it comes into contact.

The teeth or projections of the roller 126 penetrate the strip 105. This serves simultaneously to form the above mentioned perforations in the strip, and to effect thermal bonding of the film 111 to the webs 110 and 112. This takes place by virtue of the fact that fibres from web 110 are forced into the perforations, where they are bonded to the film 111 and, in some cases, to the web 112. Also material from the film 111 is forced into the web 112 and thermally bonded thereto. The bonding is a result partly of the heat from the teeth, and partly from the pressure which they apply. Bonding between the film 111 and the webs 110 and 112 the takes place only at the edges of the perforations. The effect of this limited bonding is that the elastic film is able to continue to manifest its elasticity is a direction transverse to the orientation of the fibres of the web 110. This means that, as already mentioned, the eventual product has maximum elasticity when the fibres in the web 110 are all aligned with one another, the fibres in the web 112 are all aligned with one another, and the fibres in web 110 are aligned with those in web 112.

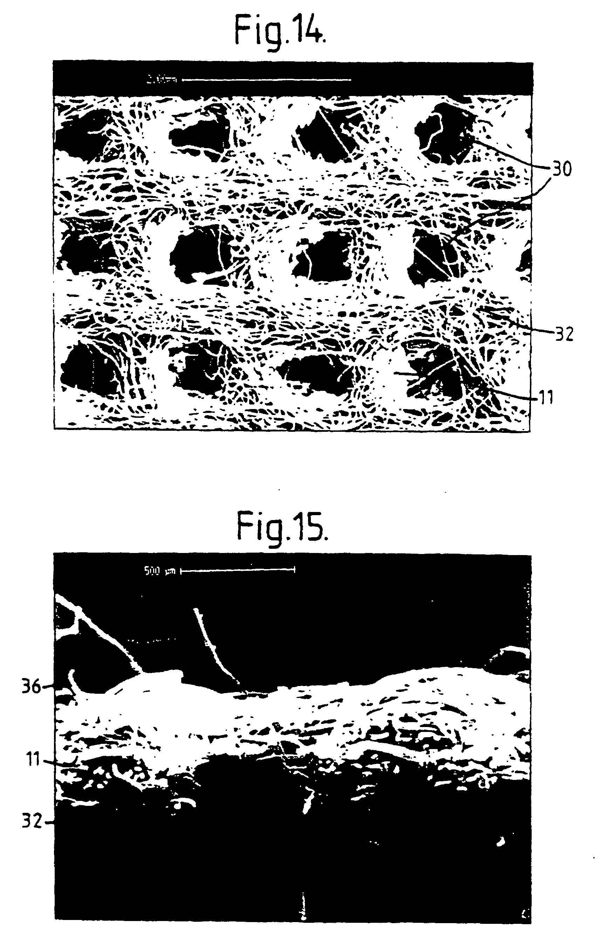

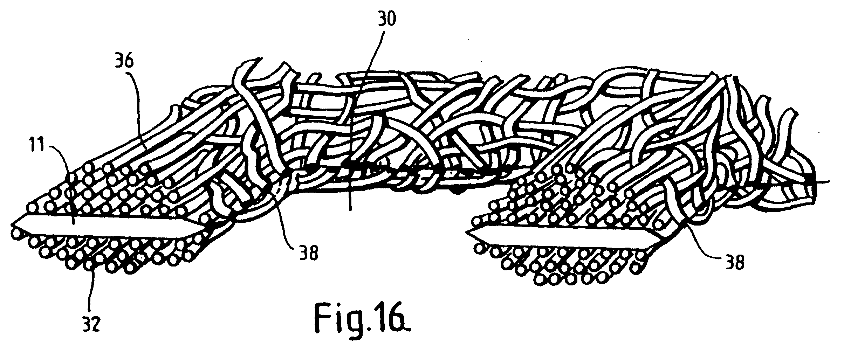

Further details of the structure according to the invention can be seen in Figures 14, 15 and 16. Figure 14 shows a square array of perforations 30 and fibres 32 of the lower fibrous layer. Also visible in Figure 14, around the perimeter of each of the perforations 130, are portions of the intermediate elastic film 111, which have been forced into the perforations in the course of manufacturing the structure. The transverse section of Figure 15, which is taken along a line passing through one of the perforations 130, shows the fibres 132, the film 111, and fibres 136 of the upper fibrous layer. Figure 16 shows substantially what is in Figure 15, but in a simplified, diagrammatic form. Reference numeral 138 indicates the area around the perimeter of each of the perforations 130 where the fibrous layers and elastic film are bonded together.

The size and spacing of the above mentioned perforations can be chosen according to the intended use of the product. However, it has been found appropriate to have the perforations arranged in a square array with approximately 7 perforations per linear cm in each direction (i.e. 49 perforations per $cm^2$), with each perforation being square and having a side length of about 0.7mm. However, it must be emphasised that other arrangements and sizes of perforations can be employed. Some are described in EP-A-207904, referred to above, to which attention is directed for details.

One embodiment of the topsheet according to this aspect of the invention will now be identified in more detail by way of example.. This comprises upper and lower webs 110 and 112 respectively, both made of polypropylene carded fibres of 1.7 dtex. The upper web has a basis weight which is higher than the basis weight of the lower web in a ratio of 1.2 :1, with the combined basis weights of the two webs being 30 $g/m^2$. The film 111 is a thermoplastic, elastomeric, styrenic block copolymer based film, 40 μm thick and with a basis weight of about 35 $g/m^2$, available from Exxon Chemical Corporation under the name EXX 500. The overall basis weight of the material is therefore approximately 65 $g/m^2$. Perforations are formed therein in a square array, with the sides of each perforation having a length of approximately 0.7mm, and with the spacing between the centres of adjacent perforations being approximately 1.4mm.

It will be understood that various modifications may be made to the embodiment just described. For example, the webs 110 and 112 may be made of fibres having some other diameter (as an example, 2.2 dtex fibres have also been used and found to be acceptable, though the results given by the 1.7 dtex were superior). Also, the ratio of the basis weight of the upper web to the basis weight of the lower web may have a value other than 1.2 :1, though it preferably is in the range 1.1 :1 to 2:1.

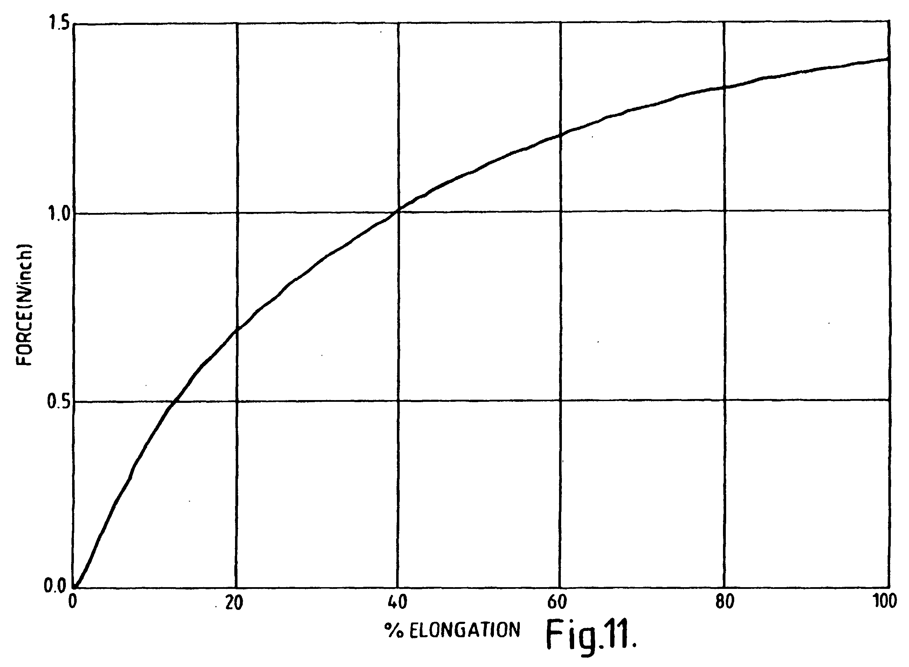

The graphs shown in Figures 11 to 13 relate to the embodiment just described. Figure 11 shows the percentage elongation achieved when a given force is applied to a rectangular sample of the material. The force is expressed as the force in N divided by the width of the sample, in inches, i.e. the distance transverse to the direction of application of the force. This shows that it was possible to elongate the material by an amount at least equal to its own length, without breaking it, and with the application of only a modest force.

Figure 11 shows the result of carrying out a hysteresis test on a sample of the embodiment. This was stretched three times to elongate it by 40% each time, with the force being relaxed between each elongation to 0.05 N/inch. This graph shows that the force required to achieve 40% elongation varied very little from one elongation to the next, and that the amount of set (i.e. permanent elongation) produced by three elongations was less than 10%.

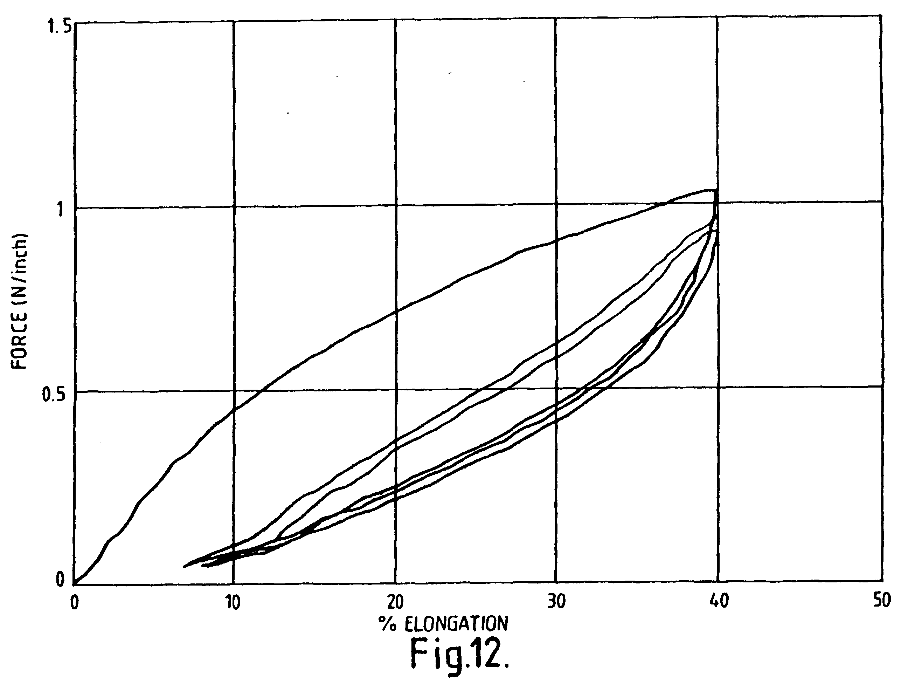

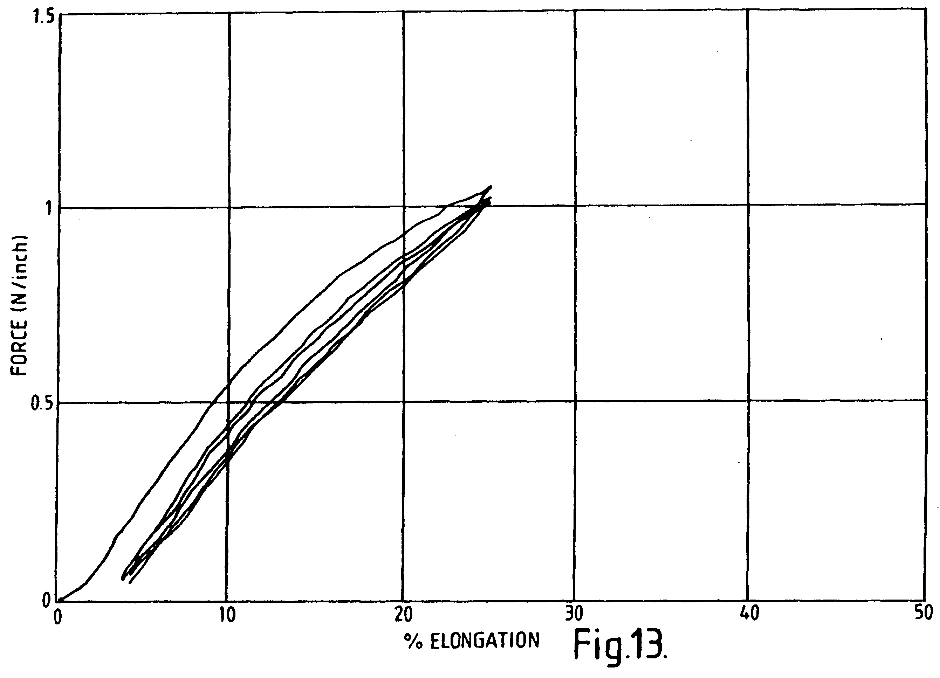

Figure 13 shows the results of carrying out a test similar to Figure 12, but on the elastic film itself. Comparison of Figures 12 and 13 shows that the elongation produced by a force of 1N/inch drops from about 40% to about 25% in going from the film by itself to the elastic product incorporating the film. This is a remarkably small drop bearing in mind that in the elastic product the elastic film is bonded to two quite substantial non-elastic webs.

The tests illustrated in Figures 11 to 13 were all carried out on samples 50mm in length and 25.4mm in width, and elongation was carried out at 100mm/min.

Tests were carried out to compare the rewetting behaviour of two forms of coversheet according to the present invention (one with hydrophilic upper and lower webs, and the other with hydrophobic upper and lower webs) with the corresponding two forms of coversheet according EP-A-207904.

In all cases the fibres of the upper and lower webs were of polypropylene. The coversheet according to EP-A-207904 had a non-elastic central film of a polyolefin material. The results of the tests are given in the following table, which also gives the thickness of the samples under a pressure of $20g/m^2$.

|  | Thickness (under $20g/m^2$ pressure) | Rewetting |
| --- | --- | --- |
| Hydrophilic Coverstock | 0.41 mm | 1.39 g |
| Hydrophilic Elasticated Coverstock | 0.37 mm | 1.02 g |
| Hydrophobic Coverstock | 0.41 mm | 0.03 g |
| Hydrophobic Elasticated Coverstock | 0.35 mm | 0.04 g |

The tests shows that the use of an elastic film as opposed to a non-elastic film has no significant effect on the rewetting behaviour of the coversheet.

Details of the method used for carrying out the rewetting test, and of the synthetic urine used in the test are given below.

Rewetting

The product is placed on an impermeable surface, and 2ml of synthetic urine is introduced into the product, which is then left for 5 minutes. Five sheets of absorbent paper, each having a weight of $220g/m^2$ are placed over the product, and a weight which exerts a pressure of 5.9 kPa on the portion of the product under the weight is placed thereon. The weight is left in position for 15 seconds. The amount of liquid absorbed by the absorbent paper is taken as the rewetting value.

Synthetic urine

The synthetic urine used was a solution in distilled water of the following salts (in weight %): Urea 2%, sodium chloride 0.9%, magnesium sulfate (heptahydrate) 0.11%, calcium chloride (anhydrous) 0.06%.

ABSORBENT CORE

For a core which is to be stretchable but not elastic, one material which can be used is a hydrophilic, stretchable non-woven material. One such material is an apertured, spunlaced material having a basis weight of 50 $g/m^2$ and consisting of 70% rayon, 30% polyethylene terephthalate (PET).

Another stretchable, substantially non-elastic material which can be used is a tissue paper consisting of conventionally CD (cross direction)creped tissue. For example, a wet laid tissue paper can be used that has been mechanically CD creped and has a weight of $47g/m^2$. Such a creped tissue is available from Aticarta, Rome, Italy. This material does have some elasticity when dry. However, it loses this when wet, and, furthermore the amount of elasticity is only small. Accordingly, it is not suitable as the sole elastic component in a construction according to the invention, all the other components being non-elastic. The construction must include at least one other component which is significantly elastic, and which retains that elasticity even when wet.

For an absorbent core which is elastic, various materials can be used. One class of suitable cores consists of cores which include materials which are inherently elastic. For example, the core can be made of a fluff mixed with a net of elastic hot melt adhesive, or a spunlaced nonwoven with an undernet of elastic hot melt. The term "net" refers to a 3-dimensional structure made of interlocked fibres of elastic hot melt adhesive mixed with cellulose fibres during the formation of the core. The term "undernet" refers to a situation where the elastic hot melt is applied as a filament, e.g. in spiral pattern, on a substrate such as a spunlaced nonwoven. Both types of structures may be carried out with equipment known in the art. Thus, "nets", of elastic hot melt fibres can be made with melt blowing equipment, e.g. the Flexi-Melt apparatus by Nordson Corporation, Georgia, USA, and a "Spyro" type applicator such as the Nordson Series 6000 by Nordson Corporation may be used to make "undernets".

A second class of suitable cores consists of cores which have been mechanically treated to make them elastic. For example, the core can be a tissue which has been creped in CD (cross direction) or MD (machine direction), or an absorbing web in which a large number of short slits have been formed.

BACKSHEET

This must be a hydrophobic material, since its function is to prevent body fluids, which are largely aqueous, reaching, and therefore soiling, the user's clothes.

For a stretchable but non-elastic backsheet, one material can be used is a hydrophobic, stretchable, spun laced, non-woven material having a basis weight of from about 30 to 40g/m$^2$, formed of polyethylene terephthalate or polypropylene fibres. This material is breathable, i.e. permeable to water vapour and other gases.

For an elastic backsheet, one material which can be used is the elastic film sold under the trade name EXX500 by Exxon Corporation and having a thickness of 40$\mu$m, as already identified above. However, this material is not breathable. Another material which can be used for an elastic backsheet is a plastic film that has been subjected to a process that provides it with elastic-like properties without attaching elastic strands to the film, and may for example comprise a formed film made in accordance with US Patents 4342314 (Radel et al) and 4463045 (Ahr et al).

ADHESIVE FOR TOPSHEET/CORE AND CORE/BACKSHEET

These two adhesives may the same as one another, though they may alternatively be different. A suitable elastomeric adhesive, which is a hot melt adhesive, is described in detail below. Briefly, this is an elastomeric hot melt adhesive composition comprising at least one thermoplastic elastomer and at least one tackifying resin, the thermoplastic elastomer(s) being a styrene/butadiene/styrene (SBS) copolymer or a blend of styrene/butadiene/styrene with styrene/isoprene/styrene (SIS) in which SIS is present in an amount equal to or less than 50& by weight of the total block copolymer, the composition being characterised in that:

(a) it is capable of bonding, when applied from the molten state, plastic and/or cellulosic materials with a 90° peel force not lower than 0.5 N/cm (as defined below);
(b) it has a tensile strength retention after 50 cycles (as defined below) of at least 40%;
(c) it has a viscosity of 120,000 cps or less at 180°C and an applied shear of 80 sec$^{-1}$.

The adhesive has elastic properties in the solid state, while its elasticity in the molten state, as shown by its essentially Newtonian behaviour at the processing (application) temperature, is very low and in many cases negligible.

Feature a) referred to above relates to the adhesive properties of the composition and in all cases the composition should have the properties of a hot melt adhesive in that it is capable of bonding appropriate substrates, typically plastic and/or cellulosic materials, when applied from the molten state. In particular, "capable of bonding" means that the composition is capable of showing adhesion on plastic and/or cellulosic materials sufficient especially for application in the construction of hygienic absorbent articles. When applied from the molten state between two substrates of plastics and/or cellulosic materials the composition gives a bond strength, measured as 90° peel of not lower than 0.5 N/cm. The composition at a weight of 5 g/m$^2$ is applied in the molten state between the substrates and 48 hours after bond formation the 90° peel strength is measured at 23°C and at a separating speed of 300 mm/min.

As described in more detail below, many compositions of the type defined above also bond appropriate substrates at room temperature and may show the properties of a pressure sensitive adhesive.

Feature b) referred to above relates to the elastic properties of the composition. The test which is used is described in more detail below and involves measuring the extent to which elastic properties are retained over 50 cycles of stretching and relaxation. The range over which the composition is stretched is related to the modulus of the composition and the likely degree of stretching of the composition in use. The figure of 40% for tensile strength retention indicates that the elastic properties of the composition are comparable with those of natural rubber and are preferably superior thereto. Preferably the tensile strength retention is at least 50%, more preferably at least 60%.

Feature c) above relates to the processability of the composition and a viscosity of 120,000 cps or less at 180°C (applied shear 80 sec$^{-1}$) indicates that the composition can be applied using conventional apparatus for use with hot melt adhesives. Preferably the viscosity is 60,000 cps or less, more preferably 30,000 cps or less. It is also highly desirable that the composition according to the invention show substantially Newtonian rheological behaviour, in particular viscosity does not vary significantly with applied shear. As discussed in more detail below, many compositions of the type defined above show Newtonian behaviour at intended processing temperatures, e.g. around 180°C.

The compositions can be formulated with any desired modulus depending on the desired end use. However the modulus has effects on the main properties of the composition and it is convenient to divide the compositions into low modulus and high modulus compositions.

Low modulus compositions are defined as compositions having a modulus of 0.5 MPa or less at 500% elongation (six times the initial length of sample) measured at 23°C under an elongation rate of 500 mm/minute. Generally low modulus compositions have a modulus in the range 0.05 to 0.5 MPa, preferably 0.05 to 0.3 MPa. Low modulus compositions generally show good adhesive properties at room temperature and may also be pressure sensitive adhesives. These compositions are usually stretched immediately after they are formed, for example after extrusion from the melt as a strip or thread. Stretching may take place immediately before or during application to an article so that the compositions are effectively applied in the stretched state. Low modulus compositions are typically used under an elongation of 400% to 1000%.

Since low modulus compositions will usually be stretched immediately after extrusion it is desirable that they should have a relatively high setting point so that they solidify quickly on extrusion. Preferably the setting point (measured by the Dynamic Mechanical Analysis method described in more detail below) is at least 80°C, more preferably at least 100°C.

High modulus compositions are defined as compositions having a modulus of greater than 0.5 MPa at 500% elongation (six times the initial length of sample) measured at 23°C under an elongation rate of 500 mm/minute. Preferably the high modulus compositions have a modulus of from 1 MPa to 10 MPa. Since pressure sensitive adhesive character is generally inversely proportional to modulus, compositions with high modulus are often applied from the molten state although some may retain sufficient pressure sensitive adhesive character to be applied at room temperature. Application from the melt implies that the composition is applied without stretching with stretching generally taking place in use and this applies particularly to compositions with a modulus of 1 MPa or higher. For this reason high solidification temperature is less critical for high modulus compositions but for convenience these are also preferably formulated to have a setting point of at least 80°C, more preferably at least 100°C. High modulus compositions are generally used at a lower degree of stretching. They are capable of giving sufficient elastic force at low deformation (typically no higher than 50%). However, in cases where the compositions retain sufficient pressure sensitive adhesive character so that they can be applied at room temperature in a stretched state, they can be used at an elongation of up to 400%.

The essential components of the composition are a thermoplastic elastomer and a tackifying resin and these will now be discussed in general terms.

Thermoplastic elastomers are a very interesting chemical and technological class of polymers which are distinguished by their characteristic behaviour. At room temperature they behave as cured rubbers showing high elasticity but in contrast to cured rubbers they can be melted and reprocessed in the same way as normal thermoplastics.

This behaviour results from a particular chemical structure. Most thermoplastic elastomers are block copolymers, i.e. their molecules are formed by blocks of different natures linked together. Different blocks can alternate along the chain as relatively short blocks (multiblock structure of the form A-B-A-B-A etc); or the molecules can have a three block structure of the form A-B-A where A are terminal blocks and B is a central block of a different nature (linear three block copolymers); or the molecules can have a "radial" or "star" structure represented as $(AB)_x$ where all midblocks B are chemically linked together at a central point and terminal blocks A are radially disposed each at the end of block B. Structures formed by only two blocks (diblocks) of the form AB are ineffective as thermoplastic elastomers in terms of their elastic behaviour.

The chemical nature of the different blocks can be varied and the resulting copolymers can be classified for example as polyurethanes, polyesters, polyethers, polyether-ester amides, etc. However, a common characteristic is the following: different blocks are physically incompatible so that they are mutually insoluble. The material can thus be considered an heterogeneous system in which different blocks, even if chemically linked in the same molecule, exist as separate entities. Blocks A of different molecules tend to associate together in microscopic regions or "domains" with the same happening for blocks B. The material so formed has an heterogeneous structure of domains A and B, each well separated, with the one present at the lower level being dispersed microscopically in the other one which constitutes a continuous phase. This continuous phase is generally formed by "soft" or rubbery blocks B which give to the material its elastic properties, while the dispersed phase A is formed by "hard" non-elastomeric blocks. Below the glass transition temperature or softening point of the hard blocks each molecule of the copolymer has its A blocks fixed in at least two points, i.e. they are "confined" in the hard domains. Accordingly, the rubbery part of the molecule can undergo stretching but without flowing relative to other molecules and when the external stretching force is relaxed it returns to its initial position for reasons of entropy.

Thus in thermoplastic elastomers, the hard blocks work as a physical vulcanization and the advantages of this processing are clear. The chemical linkages that form the vulcanized structure of a standard rubber cannot be removed by heating and at sufficiently high temperature the rubber simply begins to decompose. On the other hand, in thermoplastic elastomers heat can effectively melt the hard domains; the material can thus be melted and processed, but hard domains giving back the pseudo-vulcanization, are formed again simply by cooling the material. It is apparent from the above explanation that diblocks, which contain only one hard and one soft block, cannot contribute to elastic properties.

Diblocks can improve processability but their content in the material must be confined within certain limits so that they do not reduce elasticity to an unacceptable extent. In addition, the total amount of hard blocks is important; too low a content will give poor elastic properties (similar to an insufficiently cured rubber), whereas too high a content will make the material behave as a very hard, super-cured rubber, again with very poor elasticity.

Amongst thermoplastic elastomeric block copolymers, the so called Styrenic Block Copolymers (SBC) are well known and widely used in many applications on account of their very good properties. Styrene block copolymers as a class are described for example in Thermoplastic Elastomers: A Comprehensive Review, Legge, Holder & Schroeder (Eds), Hauser Publishers (1987), Chapters 3, 4 and 12(1). They can have the structures already mentioned above as:

- multiblock A-B-A-B-A-B- ..etc.
- linear triblock A-B-A
- radial or "star" polymers $(AB)_x$ where x > 2.

A represents a "hard" block of a vinyl-arene polymerized monomer, generally styrene or alpha-methyl-styrene; and B represents a "soft midblock" generally formed by a rubbery monomer such as poly(butadiene), (isoprene), (ethylene-butylene) or (ethylenepropylene) rubbers.

The content of diblock molecules A-B in such products can be as high as 80% by weight and in special commercial products can even form the totality of the polymer. These products are used for particular applications because, for the reasons discussed above they have no or very poor elastic properties. Diblocks can help processing and improve adhesive properties but in order to retain good elastic characteristics their content in the thermoplastic elastomeric block copolymer should be kept lower than 40% by weight.

SBC's are widely used as substitutes for vulcanized rubbers, their hardness, modulus and general mechanical and elastic properties being strongly related to the content of hard blocks, formed especially by polystyrene. They have also found use as base polymers for hot melt adhesives because of their generally good mechanical characteristics, easy tackification of their rubbery midblocks and good thermal stability which make them superior to traditional bases for hot melts such as ethylenevinylacetate copolymers. However the main object of standard compositions has been to optimize adhesive properties with retention of at least some of the elastic properties, typical of the base polymer, not being taken into account.

Thermoplastic elastomeric block copolymers known as SBC's, typically have the following characteristics:

- They are formed by two kinds of monomers each polymerized in blocks of the same monomer units, the blocks being distinct even if chemically linked inside the copolymer molecule.
  Moreover the two kinds of blocks must be mutually incompatible.
- The structure according to which the two kinds of present blocks are linked in the molecule can be:

  - alternating multiblock as .... A-B-A-B-A-B....
  - triblock linear as A-B-A
  - radial or star structure as $(A-B)_x$ where x > 2.

- "A" represents blocks of a polymer derived from a vinyl-arene monomer, typically styrene or alpha-methyl-styrene. They are called hard blocks because at room temperature these polymeric species are hard, glassy and fragile materials being under their glass transition temperature (Tg).
  Typically useful constituents for hard blocks have Tg well above room temperature and preferably higher than 90°C.
- "B" represents blocks of a rubbery polymer having a Tg < 0°C and preferably < -40°C.
  Typically these "soft" blocks are formed by rubbers such as polybutadiene and polyisoprene.

In the common technological lexicon the resulting thermoplastic elastomeric block copolymers are often referred to by the abbreviations SBS and SIS respectively. As already discussed in terms of the mechanism of the generation of elastic properties in these type of polymers, and particularly the function of hard blocks in giving a physical vulcanization to the polymer, useful SBC's contain at least two hard blocks "A" per molecule and at least one soft block "B". Molecules formed by one block of A and one block of B (the so called diblocks) should, for use in the present invention, be kept lower than 40% by weight in the base polymer.

It is widely recognised in the literature that differences exist between SIS and SBS copolymers which are relevant to the formulation of hot melt adhesives. SBS copolymers generally cost less than comparable SIS copolymers and SBS copolymers can be synthesized to exhibit better elasticity than comparable SIS copolymers. However it has not hitherto been possible to take advantage of these potentially advantageous properties of SBS copolymers as a result of the fact that SBS copolymers have not generally shown adequate adhesive properties and SIS copolymers are much easier to tackify. For this reason hot melt adhesives have generally been formulated using SIS copolymers as the predominant SBC. Both US-A-4418123 and EP-A-0424295, which are discussed above, clearly prefer SIS as the SBC on which the compositions are based and neither document discloses a composition based on SBS which has satisfactory properties.

It has been found compositions in which SBS copolymers are the main styrene block copolymer(s) can be produced with satisfactory adhesive properties whilst at the same time retaining the advantages of SBS copolymers with respect to elasticity which have been mentioned above. Thus, compared to compositions based on other SBC's, compositions can be formulated with better elastic properties, quicker elastic return, more flat stress/strain diagrams even at elongations > 1000 % and give compositions of better processability (more Newtonian rheological behaviour). However, it should be noted that direct comparison between SIS and SBS based compositions is very difficult since the compositions need to be formulated in different ways. Accordingly, it would not generally be possible to substitute an SBS copolymer for an SIS copolymer in a hot melt composition and obtain satisfactory properties and other adjustments need to be made to the formulation depending on the nature of the SBC in order to obtain optimum results. The way in which compositions should be formulated to obtain the desired properties is discussed in more detail hereinafter.

Thus the compositions according to this invention are based on SBS copolymers or a blend of SBS/SIS in which SIS is present at levels equal or less than 50% by weight of the total block copolymer.

All thermoplastic elastomers can be processed in the molten state using various technologies and in various apparatus, in all cases showing in the solid state properties similar to those of a cured rubber. Potentially all thermoplastic elastomers can be made adhesive. Some adhere well enough in the molten conditions to different substrates. However it is clearly highly desirable to obtain thermoplastic elastomers which are capable of adhering at room temperature or at only moderately elevated temperature to various substrates.

Thus, whilst pure thermoplastic elastomers have some adhesivity at high temperature, this adhesivity can be conveniently enhanced both in terms of the strength of the bonds formed with different substrates and in terms of the range of temperatures at which strong bonds are formed.

This enhancement is obtained by the use of at least one suitable tackifying resin. More particularly, much better adhesive properties and even self adhering properties at room temperature (pressure sensitive behaviour) can be obtained by blending thermoplastic elastomers with the materials known as tackifying resins which, as a class, are well known in the literature.

When thermoplastic elastomers are assembled at room temperature (e.g. because it is desired to pre-stretch them in the solid state and bond under tension) it is necessary that they exhibit the typical behaviour of true pressure sensitive adhesives and this generally requires a blend of a thermoplastic block elastomer and tackifying resin. It should be noted that in order to enhance the adhesive properties of the thermoplastic elastomer (both at high temperature and at room temperature), only the soft (rubbery) blocks of its molecule should be modified by the tackifying resin. Thus, only interactions between the soft (rubbery) blocks and a resin, substantially compatible with them, causes the generation of tack; while the eventual modification of hard blocks with a resin never leads to the development of adhesive behaviour.

Not only do the hard blocks not exhibit any adhesive activation but their eventual modification by a tackifying resin could "soften" their mechanical strength. This risks impairing their ability to function as "centers of physical vulcanization" for the elastomer, consequently destroying elastic behaviour.

So, for the various thermoplastic block elastomers, depending on the chemical nature of their soft and hard blocks, suitable tackifying resins can be identified which must be compatible (i.e. soluble and capable of creating the appropriate physical modification of the system) only with the soft or rubbery blocks, whilst compatibility with the hard blocks is as low as possible or even zero, in order to retain as much as possible of primary elastic properties of the polymer. However, the amount of tackifying resin must be controlled since the addition of quantities of tackifying resin (s), which are too large, even if the tackifying resin is compatible only with the midblocks (soft blocks) and fully incompatible with the hard blocks, could still impair the elastic properties of the resulting formulation. In any case, the addition of the resin constitutes a dilution of the concentration of the hard block domains, weakening their ability to function as centers of "physical crosslinking" for the elastomer. Thus both the content of hard domains in the base thermoplastic block elastomer and the content of the elastomer in the final formulation must be such to ensure a sufficient final concentration of hard domains in the formulation to retain appropriate levels of "physical vulcanization" and thus of elastic properties.

Therefore, on the one hand, it is important to control the final concentration of hard blocks in the composition.

On the other hand, the addition of resin(s) which are compatible only with the hard blocks and their domains, is completely ineffective in the development and/or improvement of adhesive properties. Resins compatible with the hard blocks will, by swelling the hard domains, stiffen the composition, increase modulus and (compared to similar levels of tackifying resins compatible with the midblock) will tend to increase viscosity. In a system already containing a tackifying resin compatible with the soft domains, the addition of resins compatible with the hard domains will also decrease the adhesivity. Accordingly, in general terms, only limited quantities of resins compatible with the "hard blocks" can be used without too great an impairment of the overall properties of the adhesive elastic hot melt. Generally they will only be used in special cases, for example, if an additional increase in modulus is required for some applications; or (using high softening point hard block compatible resins) if a higher setting temperature or a better temperature resistance is desired.

The compositions can be used to elasticate structures in which they are applied without the use of any glue, for example structures where elastication is obtained conventionally by elastic formed of vulcanized rubbers. One material (the adhesive elastic hot melt) can substitute for the use of two materials (the rubber and the glue to fix it) with a substantial saving in costs. Normally rubber elastics are covered with talc to prevent sticking of the elastics in the packaging. Talc can give rise to problems at the stage of adhesion with glues.

Moreover the thermoplastic, adhesive elastic hot melt can be directly extruded in varied geometrical forms directly during the construction of product which are to be elasticated. It can be extruded as strands or yarns, as bands, as films, etc. Structures such as bands or films can be also foamed before the extrusion, obtaining elasticated structure which are particularly soft. Elastication can be also applied according to non-linear (curved) geometries which makes the anatomical fitting, of the product comprising the elastication, to the wearer's body particularly good. This is very difficult to obtain with standard rubber yarns of ribbons. Under different geometrical forms the adhesive elastic hot melts can be applied both in an already stretched or an unstretched state. In the first case the extruded melt is cooled immediately after the extrusion die and stretched at the desired elongation. In this case it is advisable that it possesses the following properties:

-    a relatively high setting point so that it solidifies immediately after the extrusion and can be elastically stretched. An elastic stretching can be given only to a solid material, because any force applied to a molten or semisolid material will cause only a plastic lengthening along the direction of force without any elastic tension.
-    good pressure sensitive properties of adhesion because the adhesive elastic hot melt will contact the substrate(s) when already cold, e.g. at room temperature.

When the material is applied without any prior elastic stretching and directly contacted to the substrate(s) to which it has to adhere at the outlet of the extrusion die, pressure sensitive behaviour is less important because bonding is made when the material is still above room temperature. In this case besides the geometrical forms mentioned above, the material can be applied to the substrate(s) e.g. by spraying or fiberization or by similar processes obtaining a network of interconnected short fibers or a network of fibers having indefinite length which can have both a random or a geometrically regular network structure (e.g. each fiber can form a spiral).

All these features are particularly suitable for the elastication of hygienic, absorbent articles, although the potentialities of the compositions are clearly not limited to these applications. The use of materials applied in an already stretched form can substitute for all of the presently used rubber elastics in baby and adult diapers, in catamenials, etc. when it is desired to have parts of the product already under elastic tension offering, as a result of their extreme versatility, the possibility of new elasticated structure of practically infinite variety. In this case, another advantage of elastic, adhesive hot melts over rubber elastics is worthy of note. As will be shown in more detail below, the preferred elastic hot melt compositions have a stress/strain diagram that is much flatter than a rubber elastic, i.e. even if already under tension a further stretching (e.g. due to the movements of the wearer of the absorbent article) causes a very low increase in modulus and in the tensile strength that is perceived by the wearer. This is especially true for low modulus compositions.

Compositions that are more conveniently applied in the unstretched state are typically used to give elastic return to structures/products only when the whole final structure/product is subject to some deformation during use. Normally in this case the typical deformations that are given in use to an absorbent article are very limited, e.g. of the order of 5-50%. Accordingly, it is necessary that the adhesive, elastic hot melt contained in these structures is able to respond with a sufficient elastic return force to external stresses even at these low elongations. For these applications, it will be generally more convenient to use formulations at higher modulus.

In summary the use of thermoplastic block elastomers, in different physical forms and with different application processes, for the elastication of structures and particularly of hygienic articles, is very advantageous.

Compositions according to the present invention in the form of threads or strips and methods for the formation thereof form the subject of our co-pending application [-] (internal reference DR3.1).

The formulations of the present invention show optimum properties, typical of hot melts, ranging from compositions that can more conveniently be strongly bonded to substrates at high temperature from the melt to about 50°C, to compositions that retain a permanent strong adhesivity on most substrates even at room temperature being true pressure sensitive adhesives. Moreover the compositions are characterized by retention of distinct elastic properties from the base thermoplastic block copolymer, showing all of the typical behaviours that define an elastomeric material in the technological sense.

Thus when stretched in the solid state and when the stress is relaxed they will return quickly to their initial length with only minor permanent (plastic) deformation. The formulations preferably having a distinct pressure sensitive character, can be applied even at room temperature, both in the unstretched or preferably in the stretched state for the elastication, in different geometrical forms in various items and particularly in absorbent, hygienic products such as baby and adult diapers or adult incontinence products different from diapers or feminine catamenials. Compositions having lower pressure sensitive character will be more conveniently be applied at temperature over 50°C, in the stretched or preferably in the unstretched state for the elastication of the same structures and products. In particular when applied in the unstretched state they will work at limited extension, e.g. up to 50% (i.e. final stretched dimension = 1.5 times initial dimension).

In order to show even at these low extensions a distinct elastic return force, these formulations will generally have a higher modulus than the previous ones, the two kinds of materials being, in fact, the extremes of one field of formulations all of which are both excellent hot melt adhesives and retain excellent elastic properties, the passage from one to another being gradual.

As already indicated, compositions of the type defined above can be divided into "low modulus" and "high modulus" formulations, this distinction being based on their modulus value and on their behaviour as pressure sensitive adhesives.

There is thus a family of compositions based on at least one thermoplastic elastic block copolymer in which SBS copolymers are the main polymer(s) and at least one tackifying resin essentially compatible with the soft (rubbery) blocks of the aforementioned copolymer, the tackifying resin being used mainly to prove adhesivity, both at high and at room temperature of the aforementioned copolymer. The compositions are extrudable and in the solid state retain a distinct elastic behaviour typical of elastomers from which they are derived. As typical examples and without any limitation, these compositions can be extruded and applied in the form threads, yarns, bands, continuous films, networks of fibers both continuous or having a finite length and in which fibers have both a random orientation or a geometrically regular conformation etc.

A lower modulus generally means that adhesive materials have a more aggressive adhesivity, so that the low modulus formulation generally have higher tack typical of pressure sensitive adhesives. They are able to form very strong bonds with many substrates on simple contact even at room temperature or in any case lower than 50°C.

Ratios between hard and soft blocks in the base thermoplastic elastomeric copolymer are very important in determining the elastic behaviour and the mechanical and adhesive properties.

Generally the higher the content of hard blocks (that conventionally will be referred to as "styrene content") the higher the modulus, the more evident are elastic properties, the quicker is elastic return after relaxation of stretching but the lower is adhesivity and especially pressure sensitive behaviour. All this is true provided that the level of hard blocks does not become so high that it forms the continuous phase and the material becomes a hard and no longer elastic material.

Useful SBC's can contain from 10 to 50% of styrene by weight. However, when modified with the tackifying resin, the behaviour of the resulting composition will be clearly governed, in terms of all of the aforementioned properties, by the resulting content of styrene or of hard blocks in the compositions; so that it is determined both by the level of styrene in the base copolymer(s) and by the content of copolymer(s) in the final composition. Too low a level of final block styrene will give poor elastic properties. Too high a level will increase the modulus and decrease the adhesivity to an unacceptable extent. Increasing final styrene level in the composition by increasing the content of copolymer(s) will increase excessively the viscosity and decrease processability. So both the level of copolymer(s) in the composition and their content of styrene should be chosen to optimize the final styrene content and thus all the above mentioned properties. optimum ranges will be indicated below.

If desired the rubbery part of the SBC can itself be cured (in a similar manner to the curing of natural or synthetic rubbers) by using suitable chemical or physical means, in particular curing systems known for synthetic rubber which are not activated by heat. This will have the effect of increasing the modulus of the overall composition.

The tackifying resin is added mainly to improve adhesive properties of the base copolymer(s) even to the extent of arriving at the typical behaviour of a pressure sensitive adhesive. Moreover it improves the processability of the thermoplastic elastomer both by giving to that composition lower absolute values of viscosity (as compared to the pure block copolymer) and a rheological behaviour that, at the indicated levels of resin, is practically Newtonian, i.e. the viscosity is dependent only on temperature and does not change with applied stress, a property which is very advantageous for easy processing. It is known that SBC's which have many very interesting characteristics, may be difficult to process as a result of non-Newtonian behaviour in the molten state as pure materials. This means that not only do they show very high viscosity but also, under the influence only of temperature, they do not appear to melt even at very high temperatures near 200°C. They can even begin to thermally decompose before showing a distinct fluid state. In order to make them flow and so to be able to process them, it is necessary to apply temperature and high mechanical stress. In any case processing of pure SBC's is difficult, viscosities are high and highly dependent on the combination of temperature and applied stress.

The basic composition of SBC and tackifying resin according to the present invention are capable of giving materials which, whilst retaining very good elastic and adhesive properties, are also easily processable because of both relatively low viscosities and of practically Newtonian (or acceptable Newtonian-like) rheological behaviour. This latter property was measured as variation at constant temperature (180°C) of the viscosity under two levels of applied shear rate, 20 and 80 sec$^{-1}$. The ratio of these two viscosities is hereinafter called the "Newtonian Index" (N.I.).

An ideal Newtonian fluid should have N.I. = 1 while a pure SBC could have, in the same conditions an N.I. even > 6; i.e. the viscosity variation, only due to the variation of applied shear rate from 20 to 80 sec$^{-1}$ is more than 6 times which can cause severe problems for regular and easy processing. For easy processability it is necessary that the compositions have only limited variation of viscosity at constant temperature with variation of applied shear rate.

More particularly, it is preferred that compositions show a Newtonian or almost Newtonian rheological behaviour based on the molten material at 180°C, by comparing the viscosities under a shear rate of 20 and 80 sec$^{-1}$. Preferred compositions do not show a variation in viscosity > 50% i.e. a ratio between viscosities (N.I.) not higher than 1.5.

It has been found that the most preferred compositions based on SBS's have an almost ideally Newtonian behaviour, with an N.I. not higher than 1.05.

In order to retain sufficiently the elastic properties of the base polymer it is necessary that the tackifying resin is compatible mainly with and preferably essentially only with, the soft, rubbery blocks of the block copolymer and does not interfere to a significant extent with hard blocks. This is governed both by the chemical nature of the resin and by its molecular weight. The compatibility of the resin with the rubbery blocks and its incompatibility with the hard blocks can be measured, for example, by determining the variation of Tg of soft and hard blocks deriving from the addition of resin. In particular, incompatibility with the hard blocks is considered satisfactory if their Tg (originally at 100°C if they are formed from polystyrene) is not changed more than 15°C by the addition of 100 parts of resin to 100 parts of copolymer. Measurements of the two Tg's requires appropriate equipment. Accordingly both the experience of formulators and the technical literature of resin suppliers can be taken into account to determine which tackifying resins are chemically compatible with the soft blocks and incompatible with the hard blocks of SBC's. As used herein, the term "compatible essentially only with the soft blocks" means that a tackifying resin is compatible with the soft blocks of the copolymer and is incompatible with the hard blocks to the extent that Tg of the hard blocks is not significantly changed and more preferably decreased by no more than 15°C on admixture of 100 parts of tackifying resin to 100 parts of copolymer. Preferably the Tg of hard blocks is not decreased at all.

More specifically a suitable main tackifying resin will be chosen from the following chemical groups which have high compatibility with soft blocks of SBC's and low or no compatibility with their hard blocks;

- hydrocarbon resins
- aliphatic resins
- polyterpene resins
- terpene phenolics
- synthetic C5 resins
- synthetic C5/C9 resins
- rosins and rosin esters

as well as their totally or partially hydrogenated derivatives. They can be used as the pure resin or also in blends.

When more than one resin is used, the main tackifying resin system, defined as the essential resin/blend of resins present at least at a level of 50% of the total amount of resin, are characterised by having a softening point between 85 and 150°C and more preferably between 100 and 140°C (all softening points being measured by the well known Ring & Ball (R & B) method).

Tackifying resins having softening point < 85°C are considered to have a prevailing plasticizing effect which may in any case be important for the development of good adhesive and elastic properties but is to be distinguished from the tackifying effect. This is due to the fact that in the processing of the present elastic hot melt compositions quick setting of the material after extrusion is desirable, especially for compositions which are to be stretched before application on the substrate, which is clearly possible only with solid materials. For this reason it is desirable that the setting point of these compositions is not less than 80°C and more preferably greater than or equal to 100°C.

Setting point is most accurately determined by using the technique known as Dynamic Mechanical Analysis under sinusoidal stress, which is well known in the science and technology of polymers and adhesives. According to this technique three main rheological parameters of the material are determined as a function of temperature:

- elastic or storage modulus G'
- the viscous or loss modulus G"
- the angle δ (delta) and its tangent, being the phase shift between G' and G".

G' is higher than G" when the material is solid. When the material is fluid G" becomes higher than G'. Naturally, G' is higher at low temperature and the reverse at high temperatures. The crossing temperature between G' and G" is taken as the true rheological solidification (or melting) point of the material.

It is preferable that the crossover temperature for the composition is greater than or equal to 80°C and more preferably greater than or equal to 100°C.

The position of the crossover point is dependent on many physical parameters of the hot melt. However, it has been found that the main influence is the softening point of the main tackifying resin and a secondary influence is the content and molecular weight of the copolymer. So the tackifying resin should preferably have a softening point from 85 to 150°C and more preferably from 100 to 140°C provided that in any case the overall composition has a true rheological solidification temperature at least of 80°C and more preferably at least of 100°C.

Besides the thermoplastic elastomeric block copolymer and the main tackifying resin, compositions can contain additional components which improve specific properties. A more detailed description of the compositions and of their principal properties is given below.

For practical reasons of clarity of description, further description will relate specifically to "low modulus" and "high modulus" compositions.

The low modulus compositions are elastic, extrudable, adhesive compositions based on at least one thermoplastic elastomeric block copolymer, suitably modified by the proper addition of at least one tackifying resin essentially compatible with its soft blocks. The polymer, or at least the polymer present at the highest level, is a polystyrene/polybutadiene block copolymer. In this embodiment the compositions according to the invention have a modulus of 0.5 MPa or less, essentially from 0.05 MPa to 0.5 MPa and preferably less than or equal to 0.3 MPa; the modulus being measured at 23°C at 500% elongation (six times the initial length of sample) under an elongation rate of 500 mm/minute. Moreover the compositions have viscosities at 180°C and with an applied shear rate of 80 sec$^{-1}$ of 120000 centipoise (cps) or less and preferably 60000 cps or less and more preferably 30000 cps or less.

The low modulus compositions will typically contain from 10 to 80% by weight, and more preferably from 15 to 50% by weight, of SBC or of a blend of SBCs having the following characteristics:

- a molecular structure that can be multiblock, linear or radial (star) provided that it contains per molecule, at least two hard-blocks formed by a vinyl-arene polymer and preferably polystyrene or poly-alpha-methyl-styrene, and at least one soft or rubbery block, the soft block of the SBC, or of the SBC present at the highest level, being polybutadiene. The diblock content in the SBC(s) should be kept lower than 40% by weight.
- the aromatic content (conventionally referred to hereinafter as "block styrene content") of the SBC(s) can vary from 10 to 50% by weight and preferably from 20 to 50% by weight.

However in order to retain significant elastic properties, both the SBC(s) level in the final composition and the block styrene content thereof should be chosen so to have a final styrene content in the composition from 3 to 17% by weight and preferably from 6 to 15% by weight.

The composition also contains tackifying resin or a blend of the same essentially compatible with the soft blocks of SBC. The preferred resins belong to the chemical groups known as:

- hydrocarbon resins
- aliphatic resins
- polyterpene resins
- terpene phenolic resins
- synthetic C5 resins
- synthetic C5/C9 resins
- rosin and rosin esters

as well as their totally or partially hydrogenated derivatives thereof.

The tackifying resin/blend of resin has/have a R&B softening point from 85 to 150°C and preferably from 100 to 140°C. The level of such resin/blend of resin in the composition can be from 20 to 90% by weight. However, in a preferred embodiment the content of resin/blend of resin described above is from 30 to 55% by weight the remainder being formed by the additional components described below which enhance elastic and/or adhesive properties.

In any case both the level and the softening points of the tackifying resin/blend of resins as well as those of additional components described below will be chosen so that the final composition has a true rheological setting temperature (measured as crossing temperature of G' and G") not below 80°C and preferably not below 100°C.

It has also been found that adhesive and/or elastic and/or mechanical properties of the binary blends SBC/tackifying resin can be improved by using additional components.

Adhesive properties can be enhanced by adding limited quantities of high molecular weight rubbers such as polyisoprene, polybutadiene, polyisobutylene, natural rubber, butyl rubber, styrene/butadiene rubber (SBR) or styrene/isoprene rubber (SIR) and blends thereof. These polymers have high viscosities and, in the uncured state, have poor elastic properties. However, adding them in quantities up to 15% by weight of the formulation and using polymers with Mooney viscosities ML (1+4) at 100°C from 30 to 70, the resulting compositions show improved pressure sensitive adhesive properties whilst still retaining final viscosities within a useful range and without any detrimental effect on final elastic properties. A particularly suitable SBR is the product sold by Enichem under the trade name EUROPRENE SOL 1205 and by Fina under the trade name FINAPRENE 1205. This product is described as an SBR in which styrene is partially distributed in blocks. Of the total styrene content of 25% by weight, from 15 to 18% has a block structure and the remainder is randomly co-polymerised with the butadiene.

Plasticization of the composition can have very good effects not only on the adhesive properties and on the viscosity but can also even improve elastic behaviour by reducing the internal (molecular) frictions that dissipate elastic energy during stretching and subsequent relaxation. In general, the composition may contain up to 40% by weight of plasticizer(s).

In a preferred embodiment, the compositions contain at least one of the following plasticizers:

- up to 40% by weight of a tackifying resin with a softening point from 50 to 85°C,
- up to 20% by weight, and preferably up to 15% by weight, of a liquid hydrocarbon resin, rosin ester or polyterpene resin with a softening point not higher than 30°C,
- from 3 to 30% by weight and preferably from 5 to 15% by weight of a paraffinic or naphthenic mineral oil having an aromatic content of less than 10% by weight in order not to interfere with the styrenic domains,
- up to 15% by weight of a liquid polyisoprene or depolymerized natural rubber or polyisobutylene, polybutene or polypropylene oils and the liquid copolymers thereof, for example PARAPOL (Exxon), or LIR (from KURARAY).

The amount of plasticizer should be such that the setting temperature is not lowered beyond the limit referred to above. In a preferred embodiment the total plasticizer content in a low modulus formulation is not less than 10% by weight and not higher than 40% by weight.

In low modulus compositions, the use of aromatic resins, which have no effect on adhesive properties, which interfere with the hard blocks of SBC's and which stiffen the composition and tend to increase viscosity, is generally not desirable and the preferred level of aromatic resins is zero. However, limited quantities of an aromatic resin or a blend of aromatic resins, for example 20% by weight or less, more preferably 10% by weight or less can be used as a reinforcement for compositions which have low total styrene content (say up to 6%) or which include significant amounts of SIS, for example 30% by weight or more of SIS based on the total SBC(s). In fact SIS copolymers, especially the ones which have a styrene content < 30% by weight when diluted into the composition by resin and other additives, can show an inadequate (too low) modulus and poor characteristics of elastic return as a result both of the intrinsic lower modulus of SIS and the low concentration of styrene, acting as a physical vulcanizing agent. In this case the aromatic resin can both increase modulus to a useful level and increase the density of hard domains, that are swollen by the resin. Useful aromatic resins have a softening point from 115 to 160°C and are chemically identified as derivatives of styrene, alpha-methyl-styrene, vinyl-toluene, coumarone-indene and copolymers thereof; alkyl-aryl resins etc.

Apart from the components discussed above the compositions can contain the usual additives such as antioxidants, U.V. inhibitors, pigments and colouring materials, mineral fillers etc. Generally in a total amount up to 20% by weight.

Without limitation as to their most suitable processing and use, the low modulus formulations are typically used in the stretched state at typical extension levels of 400-1000%. They are characterized by very high elongation at break (over 1100% and often over 1400%) and very good adhesive, often pressure sensitive adhesive properties.

In order to simulate the application of the composition into an absorbent article, pressure sensitive adhesive properties were measured as loop tack (or "Quick Stick Tack") and 90° peel according to the standard methods FINAT Test Method No 9 for the loop tack and FINAT test Method No 2 for the 90° peel, modified as defined herein.

- For both tests the compositions were applied on a polyester film at a weight of 80 g/m$^2$.
- Adhesive properties, both as loop tack and 90° peel, were measured on a polyethylene film fixed on the standard test plate.
- Loop tack values were expressed as peak values, ignoring the initial peak.

- The 90° peel was evaluated after compression by a 400 g roll passed back and forth, i.e. two passes, one in each direction, and measurements were made 20 minutes after contact of adhesive and polyethylene.

The compositions according to the invention generally have a loop tack > 5 N/cm and 90° peel > 7 N/cm (separating speed = 300 mm/min). Materials which can usefully be bonded and assembled at room temperature into a hygienic product are considered to be those which show on polyethylene loop tack > 2.5 N/cm and 90° peel strength > 3 N/cm.

High modulus formulations are elastic, adhesive, extrudable compositions similar to those described previously and having the following characteristics:

1) They have a modulus higher than 0.5 MPa and more preferably not lower than 1 MPa up to 10 MPa.

2) At 180°C and with an applied shear rate of 80 sec$^{-1}$, they have viscosities of 80000 cps or less preferably 50000 cps or less and more preferably 35000 cps or less.

3) They are based on the same types of SBC's referred to previously, but which have a final block styrene content in the composition of from 15 to 30% by weight and preferably from 15 to 25% by weight.

4) The SBC or blend of SBC's which is used has a diblock content not exceeding 25% and preferably not exceeding 10%. The most preferred polymers are those with no content of diblocks such as those marketed by DEXCO Co under the trade name VECTOR.

5) The preferred SBC(s) contain from 20 to 50% by weight of styrene and the preferred level of SBC or blend of SBC's in the composition is from 35 to 75% by weight, provided that both the styrene content of SBC's and their level in the composition are such as to match the requirement of point 3) above about final styrene content.

6) The tackifying resin/blend of tackifying resins has/have the same chemical and physical characteristics as already discussed above. However, the preferred content is from 20 to 40% by weight.

7) The content of high molecular weight rubbers such as polyisoprene polybutadiene, polyisobutylene, natural rubber, butyl rubber, SIR and SBR should not exceed 10% by weight and preferably is less than 5% by weight based on the total composition.

8) The total content of plasticizers, as previously described should not exceed 25% by weight.

9) Aromatic resins or blend of the same are still preferably avoided for their detrimental effect on adhesive and stress/strain properties (steeper stress/strain diagrams; generation of a yield point and consequently of an unrecoverable plastic deformation). However, as in the previous case, these materials can be present at levels up to 20% by weight with acceptable properties in the composition provided that the non-adhesive/non elastomeric part of the composition does not exceed 50% by weight of the total composition. This non-adhesive/non-elastomeric part is formed by the sum of the total styrene content in the composition plus the content of aromatic resin/resins.

Other requirements and other possible further components and additives remain the same. In particular it is still required that the true rheological setting temperature (measured as the crossing point between G' and G") is not lower that 80°C and preferably not lower that 100°C.

Again with no limitations on their processing and use, these high modulus compositions are often applied in the unstretched state, especially the ones having moduli > 1 MPa. This preferred use is due to the fact that they are capable of giving sufficient elastic return forces even at low deformations (typically not higher than 50%) which are often met during use of stretchable hygienic articles which can conveniently be made elastic and resilient in this way. This is also due in part to the fact that assembling with the composition in the stretched state implies the need to apply the composition at about room temperature and so requires that it adhere strongly to substrates even in these conditions (pressure sensitive adhesive properties). The pressure sensitive character of adhesives tends to be inversely proportional to their elastic modulus.

However, some of the high modulus compositions still retain distinct and useful pressure sensitive behaviour (loop tack on PE > 2.5 N/cm; 90° peel on PE > 3 N/cm) and can be applied also at room temperature and in the stretched state at typical elongations up to 400% with the elongations at break of these compositions typically over 900%.

Adhesive properties are measured under the same conditions as for low modulus compositions.

The unexpectedly good level of elasticity of the compositions can be measured as retention of tensile strength after cyclic deformation. This is a test that simulates conditions in use on a hygienic article where the movements of the wearer can cause further and subsequent elongations of the elasticated parts which, for optimum behaviour, must regain their initial length with only minor losses of tensile strength. All the compositions are tested starting from an already stretched state and are given a further elongation of about 15% of the initial stretched length, in order to simulate movements of the wearer. The compositions were cyclically stretched and released fifty times from the initial elongation to the further stretched elongation.

The % retention in tensile strength at the initial elongation after 50 cycles of stretching at a speed 500 mm/minute, compared to the initial tensile strength, was taken as a measure of the elasticity of the materials. Tests were performed at room temperature on bands 2.54 cm wide.

- Low modulus compositions were stretched at an initial elongation typical of intended applications of 800% and then cyclicly further stretched and released 50 times between 800 and 920% (ie from 9 to 10.2 times the initial length of the sample).
- High modulus compositions were tested in the same manner but at an initial elongation typical of intended applications of 300% and under 50 cycles of further elongation and relaxation between 300 and 345%.

The term "tensile strength retention after 50 cycles" as used herein refers to the test described above. A natural rubber vulcanized elastic produced by the company JPS Elastomerics which is used in the leg elastication of diapers and applied at an initial stretched deformation of 220% was taken as a reference and was cyclicly deformed 50 times between 220 and 255% It was found that under these conditions the natural rubber vulcanized elastic, after 50 cycles, had an average retention of tensile strength equal to 47% of the initial tensile strength at 220%. This level of retention of tensile strength was considered indicative of good elastic behaviour.

More generally it was observed that materials that do not lose more that 60% of their initial tensile strength in these test conditions, show good elastic behaviour. Retention of tensile strength less than 40% represents unsatisfactory elasticity as indicated by slow return to the initial length after release of stretching, high permanent plastic deformations etc.

As will be shown in the following examples, both low and high modulus compositions show good elastic behaviour, at the same level or better than the natural rubber vulcanized elastic.

More specifically high modulus compositions retained up to 67.5% of their initial tensile force and low modulus compositions up to 59.8%.

Elastic properties were also judged by the following method: The compositions in the form of bands 2,54 cm wide, were tested at 23°C and at a stretching speed of 1000 mm/minute, with 3 cycles of elastic hysteresis between zero and a typical possible elongation in application i.e. 800% for low modulus and 300% for high modulus compositions. The elastic energy of each cycle evaluated as the area of the cycle was recorded and the ratio between the elastic energy of the third and the first cycles was determined as retention of elastic energy after 3 hysteresis cycles. For good elastic behaviour it is believed that under these test conditions, a retention of elastic energy not lower than 30% is required.

Some examples of compositions of the type defined above will now be given. In the case of proprietary products, details of their nature and composition is that provided by the manufacturer.

The compositions of all of Examples 1 to 5, when applied from the molten state between plastics and/or cellulosic materials at a weight of 5g/m$^2$ showed a bond strength well in excess of 0.5 N/cm measured as 90° peel.

## EXAMPLE 1

An SBC polymeric system based on SBS (styrene-butadiene-styrene block copolymers) was formulated as follows:

| CARIFLEX TR-4113 S | 36% by weight |
|---|---|
| EUROPRENE SOL 1205 | 8% |
| DERCOLYTE A 115 | 45.8% |
| FORAL 85-E | 6% |
| HERCOLYN D-E | 4% |
| IRGANOX 1010 | 0.2% |

where:

- CARIFLEX TR-4113 S is an oil-extended SBS copolymer available from SHELL Co said to contain: 68.5% by weight of a linear triblock SBS having a styrene content of 35% by weight and with a diblock content < 20% by weight 31.5% by weight of a naphthenic mineral oil, acting as a plasticizer, and containing less than 5% of aromatics.
- EUROPRENE SOL 1205 is a styrene/butadiene rubber (SBR) available from ENICHEM. (The product FINAPRENE 1205 available from FINA is similar and could equally be used.) It is described as a solution polymerised SBR having a Mooney viscosity ML (1+4) at 100°C equal to 47 and a total styrene content of 25% by weight. This styrene is partially (typically from 15 to 18%) distributed in blocks with the remainder being randomly copolymerized with butadiene. This randomly copolymerized styrene gives the rubbery part of the molecule the chemical structure of an amorphous SBR, which contributes to the development of particularly good pressure sensitive adhesivity.

- DERCOLYTE A 115 (the main tackifying resin) is available from DRT. It is a polyterpene resin derived from alpha-pinene having a softening point of 115°C.
- FORAL 85-E is a tackifying resin composed of a hydrogenated glycerol ester of rosin available from HERCULES Co. It has a softening point of 85°C.
- HERCOLYN D-E is a liquid methyl ester of rosin available from HERCULES.
- IRGANOX 1010 is a phenolic antioxidant available from CIBA-GEIGY.

The composition was found to have the following properties:

- total styrene content = 10.6% by weight of which 10.1% is in blocks
- viscosity at 180°C at 80 sec$^{-1}$ = 20520 cps
- modulus at 500% elongation - 0.182 MPa (low modulus)
- elongation at break > 1400% (1400% was the maximum elongation achievable on the machine used for this determination)
- rheological setting temperature (crossover point of G' and G") = 125°C
- loop tack on PE = 8.5 N/cm
- 90° peel on PE = 16.3 N/cm
- tensile strength retention after 50 cycles between 800 and 920% = 59.8%
- elastic energy retention after 3 hysteresis cycles between zero and 800% = 57.7%
- Newtonian Index (N.I.) = 1.05.

The composition showed extremely good elastic and adhesive properties and was considered completely suitable for elastication of structures, particularly hygienic absorbent articles. It was easily processable i.e. extrudable, having quick setting (stretchable on line) and having good pressure sensitive characteristics allowing the formation of strong bonds on simple contact with many substrates even at room temperature.

## EXAMPLE 2

The formulation was:

| | |
|---|---|
| CARIFLEX TR-4113S | 38.8% by weight |
| FINAPRENE 1205 | 8.9% |
| DERCOLYTE A115 | 26% |
| FORAL 85-E | 26% |
| IRGANOX 1010 | 0.3% |

The following properties were measured:

- total styrene content = 11.5% by weight of which 10.9% is in blocks
- viscosity at 180°C at 80 sec$^{-1}$ = 28180 cps
- modulus at 500% elongation = 0.188 MPa (low modulus)
- elongation at break > 1400%
- rheological setting temperature = 120°C
- loop tack on PE = 8.6 N/cm
- 90°C peel on PE = 10.4 N/cm
- tensile strength retention after 50 cycles between 800 and 920% = 59.1 %
- elastic energy retention after 3 hysteresis cycles between zero and 800% = 48.3 %
- Newtonian Index (N.I.) = 1.01.

The composition was similar to that of Example 1, the main variation being the fact that about 50% of the high softening point tackifying resin was substituted by a lower softening point resin and the only plasticizer was the oil contained in CARIFLEX TR-4113 S (12.2% by weight of the composition). Nevertheless it was found that the composition still retained a very high solidification temperature, quick setting as well as optimum elastic and pressure sensitive adhesive properties and excellent processability, so that it was easily possible to extrude and immediately stretch it even to 800% in the form of very thin threads (diameter = 0.4 mm) that could be applied for the side elastication of an absorbent product.

## EXAMPLE 3

A different system based on radial SBS was tested, more precisely a blend of one SBS with relatively low hard block content and one SBS with relatively high hard block content. The formulation was as follows:

| FINAPRENE 415 | 17.7% by weight |
|---|---|
| FINAPRENE 401 | 7.0% |
| FINAPRENE 1205 | 8.0% |
| ZONATAC 115 LITE | 45.8% |
| FORAL 85-E | 6.3% |
| HERCOLYN D-E | 4.0% |
| SHELLFLEX 4510 FC | 11.0% |
| IRGANOX 1010 | 0.2% |

where:

- FINAPRENE 415 and FINAPRENE 401 are radial SBS copolymers available from FINA. Both are supposed to contain less than 20% diblock and to be formed by a "star" structure of four blocks of SB chemically linked in a central point through the butadiene blocks. FINAPRENE 415 contains 40% by weight of block styrene and FINAPRENE 401 contains 22% of block styrene.
- ZONATAC 115 LITE is a hydrocarbon modified terpene tackifying resin with a softening point of 115°C available from Arizona Co. It is supposed to be based on limonene modified with styrene.
- SHELLFLEX 4510 FC is a naphthenic mineral oil, available from SHELL, which is supposed to have an aromatic content < 5%.

The following properties were measured:

- total styrene content = 10.6% by weight of which 10.1% is in blocks
- viscosity at 180°C at 80 sec$^{-1}$ = 12810 cps.
- modulus at 500% elongation = 0.223 MPa (low modulus)
- elongation at break > 1300%
- rheological setting temperature = 107°C
- loop tack on PE = 6.4 N/cm
- 90° peel on PE = 14 N/cm
- tensile strength retention after 50 cycles between 800 and 920% = 50%
- elastic energy retention after 3 hysteresis cycles between zero and 800% = 44.9%
- Newtonian index (N.I.) = 1.04.

The composition showed properties typical of a very good and easily processable elastic, extrudable adhesive material.

## EXAMPLE 4

The following high modulus composition was made with the formulation:

| VECTOR 4461-D | 44.8% by weight |
|---|---|
| ZONAREZ 7115 LITE | 37% |
| FORAL 85-E | 5% |
| ZONAREZ ALPHA 25 | 3% |
| PRIMOL 352 | 10% |
| IRGANOX 1010 | 0.2% |

where:

- VECTOR 4461-D is a linear SBS copolymer having 43% by weight of styrene and non diblock content available from DEXCO Co.

- ZONAREZ 7115 LITE is a polyterpene tackifying resin, having a softening point of 115°C, derived from limonene, available from ARIZONA Co.
- ZONAREZ ALPHA 25 is a liquid tackifying resin (S.P. = 25°C) derived from alpha-pinene having a very good plasticizing effect. It is available from ARIZONA Co.
- PRIMOL 352 is a plasticizing, paraffinic mineral oil available from EXXON, which is said to contain non aromatics.

The following properties were measured:

- total block styrene content = 19.3% by weight
- viscosity at 180°C at 80 sec$^{-1}$ = 16810 cps.
- modulus at 500% elongation = 1.07 MPa (high modulus)
- elongation at break = 987%
- rheological setting temperature = 111°C
- loop tack on PE = 4.3 N/cm
- 90° peel on PE = 6.5 N/cm
- tensile strength retention after 50 cycles between 300 and 345% = 67.5%
- elastic energy retention after 3 hysteresis cycles between zero and 300% = 63.3%
- Newtonian Index (N.I.) = 1.05.

The composition was a good elastic material useful especially at low elongations. It showed acceptable semi-pressure sensitive characteristics so that is can be bonded to materials also at room temperature in the stretched state.

### EXAMPLE 5

The formulation was:

| VECTOR 4461-D | 54.8% by weight |
|---|---|
| ECR 368 | 35% |
| PRIMOL 352 | 10% |
| IRGANOX 1010 | 0.2% |

where:

- ECR 368 is a hydrogenated hydrocarbon tackifying resin, available from EXXON and having a softening point of 100°C.

The following properties were measured:

- total block styrene content = 23.56% by weight
- viscosity at 180°C at 80 sec$^{-1}$ = 34000 cps.
- modulus at 500% elongation = 1.61 MPa (high modulus)
- elongation at break = 947%
- rheological setting temperature = 114°C
- loop tack on PE = 1.3 N/cm
- 90° peel on PE = 2.6 N/cm
- tensile strength retention after 50 cycles between 300 and 345% = 62.3%
- elastic energy retention after 3 hysteresis cycles between zero and 300% = 38.3%
- Newtonian Index = 1.05. ,

The composition was made so to maximize modulus whilst still retaining acceptable elasticity and good adhesivity at temperatures higher than room conditions. Such a material is more conveniently applied in the unstretched state and bonded directly at temperature > 50°C. It gives good elastic return forces even at very low extensions, e.g. modulus at 20% elongation = 0.236 MPa. However, it also works well in the stretched state, e.g. 300% elongation, and shows adhesive properties on PE which are not negligible even at room temperature.

### EXAMPLE 6

This example relates to the stress/strain diagrams of the compositions of Examples 1 to 5. The elastic hot melt compositions should desirably have a stress/strain diagram that is much flatter than a rubber elastic, i.e. even if already under tension further stretching (e.g. due to the movements of the wearer of the absorbent article) cause only a very low increase in modulus and in the tensile strength that is perceived by the wearer. This is especially true for low modulus compositions and can be seen by measuring the average increase in modulus for a given extension. Low modulus compositions, which are typically applied in the already stretched state, were judged as the mean increase in modulus per 100% increase in elongation, by dividing by 8 the total increase in modulus between 0 and 800% elongation (nine times the initial length).

Referring to the low modulus compositions mentioned in the above Examples the results were as follows:

| EXAMPLE NO. | MEAN INCREASE IN MODULUS PER 100% STRETCHING |
|---|---|
| 1 | 0.044 MPa/100% stretching |
| 2 | 0.045 MPa/100% stretching |
| 3 | 0.063 MPa/100% stretching |

The high modulus compositions of Examples 4 and 5, which are generally used in the unstretched state or in any case at lower elongations, were judged as mean increase in modulus per 100% increase in elongation between zero and 300% final elongation:

| EXAMPLE NO. | MEAN INCREASE IN MODULUS PER 100% STRETCHING |
|---|---|
| 4 | 0.169 MPa/100% stretching |
| 5 | 0.284 MPa/100% stretching |

To adhere the topsheet to the core, the adhesive is applied in discrete areas only, so that the fluid entering the pantiliner through the topsheet is able to pass into the core. For example, the adhesive may be applied in lines at a rate of 0.06g/linear m. Other patterns in which the adhesive can be applied include free-window coating (i.e. coating only part of the surface, so as to leave at least one adhesive-free zone, or window, spaced from the outer edge of the topsheet and core), and full coating with a low enough basis weight (maximum 15 g/m$^2$) that liquid still gets through to the core.

To adhere the core to the backsheet, a continuous layer of adhesive may be applied if desired, for example at a rate of 20g/m$^2$, though it may be applied only in discrete areas, if that is preferred, for example for reasons of economy.

Backsheet-to-panty adhesive

An adhesive used for this purpose is referred to for short as a PFA (panty fastening adhesive). One suitable PFA is that supplied under the trade name SAVARE LA 203 by Savarè of Milan, Italy. This adhesive is based on an SEBS (styrene-ethylene-butylene-styrene) copolymer and has a softening point of 65°C according to the method of ASTM E28-67.

The PFA is preferably applied in particular and two classes of such patterns will now be described. Briefly, what are called herein Class 1 patterns are ones in which the adhesive is applied to at least one surface region adjacent the periphery of the article, and Class 2 patterns are ones in the adhesive is stretchable and may be applied over the whole, or at least a major part of the surface of the article.

The adhesive patterns are described below in the context of their use on pantiliners according to the invention. It is to be understood, however, that the invention is also applicable to sanitary napkins, incontinent pads, and other articles which are designed to absorb and retain liquid and other discharges from the human body.

The use of panty fastening adhesive is well known and attention is directed particularly to International Patent Publications Nos. WO 92/04000 (Papa et al) and WO 93/01785 (Osborn et al) which include descriptions of various patterns in which the panty fastening adhesive may be applied.

It will be appreciated that the pantiliners according to the invention are highly flexible and, because of their elasticity, are potentially able to follow the stretch of the panty in use. However, these very characteristics pose potential problems in relation to the panty fastening adhesive. The adhesive connection between the panty and the pantiliner must be such that it does not impair the elastic properties of the pantiliner, or at least does not do so to any significant extent. It is an object of aspects of the invention which are about to be described to provide adhesive patterns which are able to deal with these problems. It should be noted, however, that the adhesive patterns described herein are also applicable to non-elastic adhesive articles, and have advantages in relation to these also. This is to deal with the fact that there is a tendency for the panty movement to cause bunching of the pantiliner so that it tends to separate itself from the panty, and to curl and stick to itself.

According to the first of these aspects of the present invention there is provided an absorbent article for adhesive attachment to an undergarment, wherein adhesive is applied to a surface of the article, said surface being outwardly defined by a perimeter, the adhesive being applied only to at least one portion of the said surface of at or adjacent the said perimeter, the remainder of the surface being substantially adhesive-free. Such adhesive patterns are referred to for convenience above as Class 1 patterns.

Preferably at least 25% of the said surface is adhesive-free, and more preferably at least 50%.

Preferably, the adhesive extends completely to the perimeter or, if there is a gap between the edge of the adhesive and the perimeter, the gap is not more than 3mm, and more preferably from 1 to 2mm in width.

In some embodiments, the adhesive is applied in the form of one or more strip-shaped regions which extend around at least a major portion of the periphery of the article, at or adjacent the periphery.

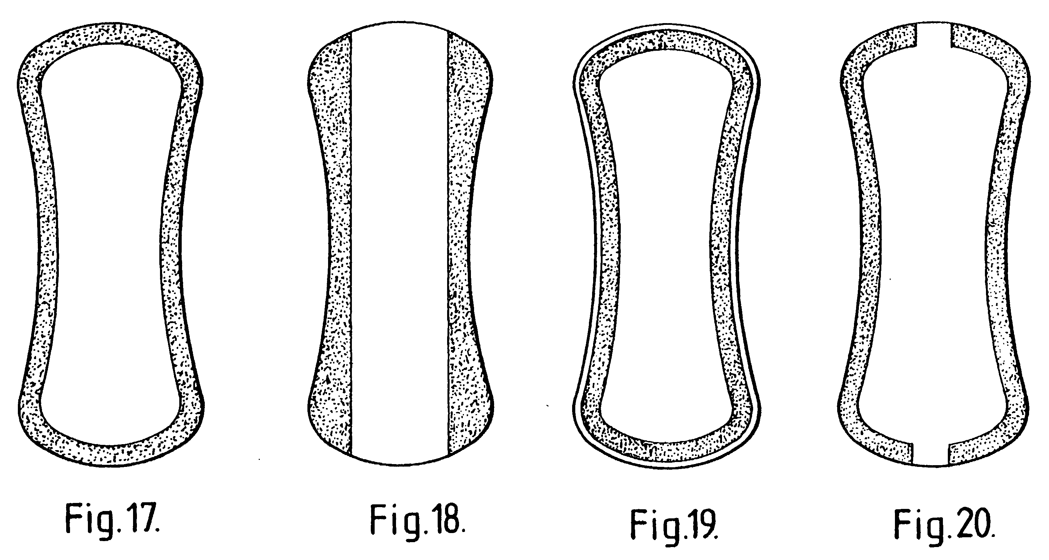

Some embodiments of this aspect of the invention will now be described with reference to Figures 17 to 23 of the accompanying drawings, which are plan views of pantiliners of hourglass shape, showing that face of the pantiliner, i.e. the external face of the backsheet, which carries the panty fastening adhesive.

Each of Figures 17 to 23 shows a pantiliner of the same shape and size. In one embodiment, it has a length of about 153mm, a maximum width of about 67mm, and a minimum width of about 51mm. It is to be understood that the pantiliner could have other dimensions and/or be of some other shape, e.g. rectangular.

The panty fastening adhesive is applied only to one or more regions of the backsheet which are adjacent the periphery thereof. A substantial portion of the backsheet, preferably at least 25% thereof, and more preferably at least 50%, has no adhesive applied thereto. As supplied to the user, the face of the backsheet to which the panty fastening adhesive is applied is covered by a protective release layer but this is removed by the user prior to use.

In the case of Figure 17, the adhesive is applied in a narrow perimetral strip the width of which is preferably from 4mm to 10mm, and more preferably about 5mm. In the case of Figure 18, the adhesive is applied in the form of two longitudinally extending strips, one extending along each lateral edge. There is thus a central strip which is free of adhesive, and which is preferably at least 15mm wide, more preferably from 29mm to 43mm wide, for example about 35mm wide. Given the overall dimensions of the pantiliner set out above, an adhesive-free strip 35mm wide thus corresponds to having two adhesive strips, each with a maximum width of 16mm and a minimum width of 8mm.

The adhesive pattern of Figure 19 is the same as that of Figure 17, except that a narrow gap, which is not more than 3mm, and is preferably from 1 to 2mm, is left adhesive-free between the perimeter of the pantiliner and the outer edge of the adhesive strip. The presence of this gap makes it easier for a user to grasp the edge of the pantiliner when the time comes to remove it from the panty. It also makes it easier for the user to remove the protective silicone-coated film by which the adhesive-coated side of the pantiliner is normally covered in the condition in which it is purchased by the user.

A similar effect is achieved with the embodiment of Figure 20, where, instead of having a continuous, narrow, adhesive-free area around the entire perimeter of the pantiliner, the adhesive strip has two breaks in it, one at each end, the breaks being preferably from 15mm to 35mm in length, and more preferably 20mm in length.

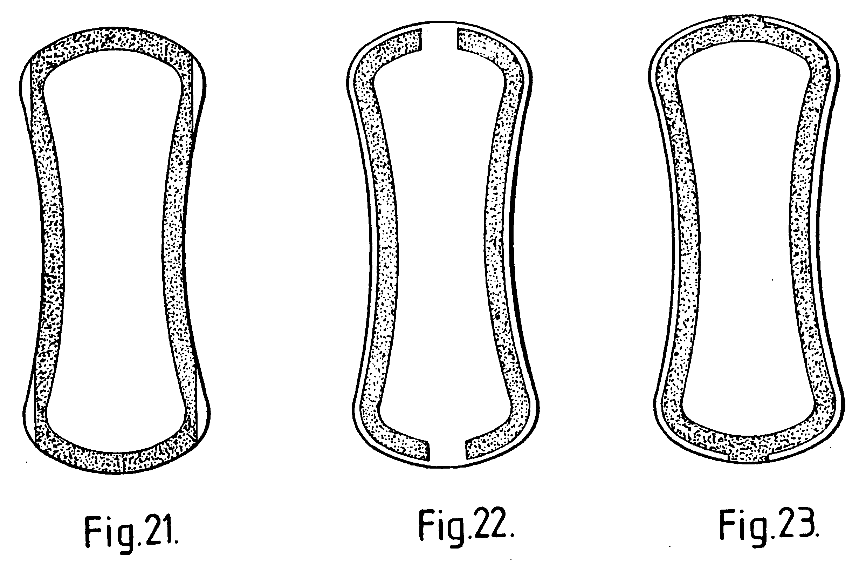

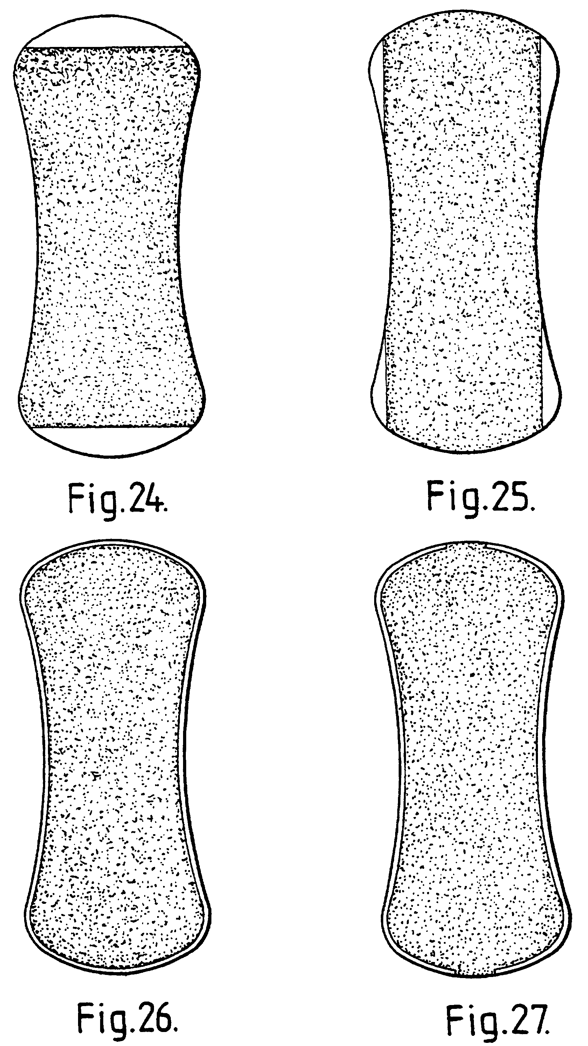

Figures 21 to 23 show some further possible adhesive patterns. Figure 21 is similar to Figure 17, but has small adhesive-free areas at each of its four corners. Each of these areas is preferably from 1 to 2mm in width at the point where its width is greatest. Figure 22 combines the adhesive-free periphery of Figure 19 with the adhesive-free gaps of Figure 20. Figure 23 is also similar to Figure 19, except that it has a small area at each end where the adhesive extends completely to the edge of the pantiliner. It will be understood that many other variations in the adhesive pattern are possible within the scope of this aspect of the present invention. Using the adhesive patterns shown in Figures 16 to 23, the pantiliner is attached to the panty over what is preferably only a small part of its area.

It will be seen that in each of the patterns shown in Figures 17, 18, 20 and 21, the adhesive extends to the very edge of the pantiliner over at least a major portion of the perimeter thereof, i.e. more than 50%, preferably more than about 65%. In the case of the pattern of Figure 17 the proportion is 100%. In the case of Figure 18 the proportion is preferably from 75% to 90%, and more preferably from 80% to 85%. In the case of Figure 20 the proportion is preferably from 80% to 95%, and more preferably from 85% to 95%. In the case of Figure 21 the proportion is preferably at least 95%.

In the case of the patterns of Figures 19 and 22, the adhesive does not extend to the very edge of the pantiliner, but extends to a point very close to that edge over the whole (Figure 19), or substantially the whole (Figure 22) of the perimeter. In the case of Figure 23 the adhesive extends to a point very close to the edge of the pantiliner over substantially the whole of the perimeter, and actually to the edge over a small portion of the perimeter.

It has been found that the provision of adhesive only at or adjacent a major portion of the periphery of the pantiliner, enables the pantiliner to retain a secure grip on the panty during movement of the latter, and avoids, or at least reduces, the tendency of the pantiliner to bunch and wrinkle and, not infrequently, stick to itself. The effect thus provided is particularly advantageous in the context of an elastic pantiliner, such as the very thin elastic pantiliner described above. In those embodiments of the invention where the adhesive is applied to a relatively small area (preferably not more than 50% of the total surface are), the adhesive used need not be stretchable, even though the pantiliner is stretchable, and this in itself represents an advantage.

Suitable panty fastening adhesives which can be used include SAVARE LA203 (produced by Savarè I.C.S. r.l.) and KORAMELT 869 produced by Kömmerling Chemische Fabrik K.G.. Both of these are SEBS (styrene-ethylene-butylene-styrene block copolymer based, hot melt adhesives.

The adhesive is preferably applied with a basis weight of about $24g/m^2$ by coating, for example using a slot die coater, or by spraying or by printing.

The description thus far has largely dealt with articles which are elastic. However, as mentioned briefly above, the adhesive patterns according to this aspect of the invention are also advantageous when applied to non-elastic articles.

Firstly, articles, for example pantiliners, which are stretchable but non-elastic, give rise to similar problems of bunching and wrinkling as do elastic articles, and these may indeed be even more acute. Accordingly, the patterns according to this aspect of the invention may advantageously be applied to stretchable but non-elastic articles.

Secondly, whether the articles are elastic, stretchable but non-elastic, or non-stretchable, it is desirable that, where they are adhesively fastened to a panty, they should be gas-permeable, though liquid-impermeable, so as to allow water vapour to pass through them. To this end, not only must the backsheet be gas-permeable, but its permeability must not be too greatly impaired by the panty fastening adhesive, an effect which is achieved by the above described patterns.

The second class of panty fastening adhesive patterns mentioned above will now be described, and these too represent an aspect of the invention. As with the first class, they will be described herein with reference to a pantiliner, preferably the pantiliner described above, but they too can be used with the other articles mentioned in relation to the first class of patterns.

According to this aspect of the present invention there is provided a stretchable, absorbent article for adhesive attachment to an undergarment, wherein a stretchable adhesive is applied to a surface of the article, said surface being outwardly defined by a perimeter, the adhesive being applied to a major portion of the said surface, and extending at least substantially to the said perimeter over a major portion of the said perimeter.

Preferably, the adhesive extends completely to the perimeter or, if there is a gap between the edge of the adhesive and the perimeter, the gap is not more than 3mm, and more preferably from 1 to 2mm in width.

Some embodiments of this aspect of the invention will now be described with reference to Figures 24 to 27 of the accompanying drawings, which are plan views of pantiliners of hourglass shape, showing that face of the pantiliner; i.e. the external face of the backsheet, which carries the panty fastening adhesive.

Each of Figures 24 to 27 shows a pantiliner of the same shape and size. In one embodiment, it has a length of about 153mm, a maximum width of about 67mm, and a minimum width of about 51mm. It is to be understood that the pantiliner could have other dimensions and/or be of some other shape, e.g. rectangular. As supplied to the user, the face of the backsheet to which the panty fastening adhesive is applied is covered by a protective release layer, but this is removed by the user prior to use.

Since the adhesive covers substantially the whole surface of the pantiliner, the panty fastening adhesive must be stretchable, in the sense that if the pantiliner stretches then so must the adhesive, without the adhesive breaking.

In each of Figures 24 to 27 a small area of the backsheet is left free of adhesive. As will be seen, in the case of Figure 24 the adhesive-free areas are at the ends. The adhesive-free end portions each preferably extend a distance from 2mm to 4mm, more preferably about 3mm, from the end of the pantiliner. In Figure 25 the adhesive-free areas are at the corners. The width of each of the four adhesive-free portions, i.e. the distance from the lateral edge of the adhesive to the point at which the pantiliner has its greatest width, is preferably from 2mm to 4mm, more preferably about 3mm. In Figure 26 there is a narrow-adhesive free strip running around the perimeter of the backsheet. The width of the adhesive-free strip is preferably less than 3mm, and more preferably from 1 to 2mm. The pattern shown in Figure 27 is the same as that shown in Figure 26, except that the adhesive-free strip is broken at opposite ends of the pantiliner by adhesive which extends to the very edge of the pantiliner.

It will be seen that in each of the patterns shown in Figures 24 and 25, the adhesive extends to the very edge of the pantiliner over at least a major portion of the perimeter thereof, i.e. more than 50%, preferably more than about 65%. In the case of Figure 24 the proportion is preferably from 75% to 90%, more preferably from 80% to 85%. In the case of Figure 25 the proportion is preferably from 70% to 90%, more preferably from 75% to 85%.

In the case of the patterns of Figures 26 and 27 the adhesive does not extend to the very edge of the pantiliner (except over a short distance in the case of Figure 27), but extends to a point very close to that edge over the whole of the perimeter.

It has been found that the provision of a stretchable adhesive at or adjacent the perimeter of a stretchable pantiliner over a major portion of the perimeter, and over a major portion of the total area of the pantiliner, enables the pantiliner to retain a secure grip on the panty during movement of the latter, and avoids, or at least reduces, the tendency of the pantiliner to bunch and wrinkle and, not infrequently, stick to itself. The effect thus provided is particularly advantageous in the context of an elastic pantiliner, such as the very thin elastic pantiliner described above.

Suitable panty fastening adhesives which can be used include those already mentioned for the Class 1 patterns, namely SAVARE LA203 (produced by Savarè I.C.S.r.1.) and KORAMELT 869 produced by Kömmerling Chemische Fabrik K.G.. The adhesive is preferably applied with a basis weight of about $24g/m^2$ by coating, for example using a slot die coater, or by spraying or by printing.

It is desirable that, where a pantiliner is adhesively fastened to a panty, it should be gas-permeable, though liquid-impermeable, so as to allow water vapour to pass through it. To this end, not only must the backsheet be gas-permeable, but its permeability must not be too greatly impaired by the panty fastening adhesive.

Tests were done to determine the permeability to water vapour of various pantiliner materials, including material from two known pantiliners, and the permeability of a sample of material from the crotch of a cotton panty. The results were as follows:

| 1. | Sample from pantiliner according to the invention, with PFA (panty fastening adhesive) pattern of Fig. 1 @ $24g/m^2$: $980g/m^2$.day. |
|---|---|
| 2. | Sample from same pantiliner but without any PFA: $1407g/m^2$.day. |
| 3. | Carefree Light New Sensitive: $918g/m^2$.day. |
| 4. | Always Pantiliner: $150g/m^2$.day. |
| 5. | Cotton panty crotch: $2300g/m^2$.day. |

The pantiliner which constituted item (1) above consisted of the following layers:

| Topsheet | Elastic covering structure as described above with reference to Figures 10 to 16, 60 $g/m^2$ |
|---|---|
| Topsheet/core adhesive | Savarè PM 17 all over coated, 10 $g/m^2$ |
| Core | Spunlaced nonwoven 70% rayon/30% PET, 50 $g/m^2$ |
| Core/backsheet adhesive | Elastic hot melt elastomeric adhesive as described in detail above all over coated 20 $g/m^2$ |
| Backsheet | Spunlaced nonwoven 100% PP, 40 $g/m^2$ |
| PFA | Savarè LA 203, 24 $g/m^2$ |
| Release layer | Silicone coated paper, 45 $g/m^2$ |

In the case of item (3) above, the adhesive is in the form of three parallel stripes extending along its length. In the case of item (4), the adhesive is applied in a single stripe 40mm wide which extends along the length of the product and almost reaches the welded edges thereof.

It will be seen that using the adhesive at a relatively low basis weight ($24g/m^2$), the reduction in permeability to water vapour produced by the adhesive was only about 30% (comparing items (1) and (2), and the resulting value was still higher than the known product of item (3).

The method by which the permeability to water vapour was determined was as follows. A sample of the product concerned, measuring 50mm x 50mm, (taken, in the case of item (1) from the centre of the pantiliner, and thus completely coated with adhesive) was mounted over the open end of a cylindrical container having a height of 30mm and an internal diameter of 26mm, containing 5ml of distilled water. The test apparatus is held at a temperature of $23°C \pm 2°$, and at a humidity of $50\% \pm 2\%$. Air is then caused to flow over the exposed surface of the sample at a rate of 2.5 to 3m/s. The water vapour permeability (WVP) measured in $g/m^2$.day is then calculated from the following equation

$$WVP = \frac{P_0 - P_{24}}{Sc} \times 10^6$$

$P_0$ = weight at time 0 hours
$P_{24}$ = weight at time 24 hours
$Sc$ = area of sample exposed to air flow.

A number of embodiments of pantiliner according to the invention are described below. Before doing so, however, a description will be given of the way in which certain relevant parameters are measured. These are stretch, elasticity, wet through, flexibility and thickness. Mention will also be made of the synthetic urine used in some of the tests.

Load

The details of this test are set out later in this specification.

Elasticity/Pad set

The details of this test are set out later in this specification.

Wet through

This test measures the amount of liquid (synthetic urine) which will pass through a product which is permeable to air and water vapour, but substantially impermeable to liquid, under certain conditions. The test is carried out as follows:

(a) Place the product on a sheet of blotting paper (220 g/m$^2$), add 2 ml of the liquid in the centre of the product at a flow rate of approximately 1 ml/10sec, and leave for 2 minutes.
(b) Place on the product a 3000 g weight having a lower surface 100 x 50 mm, whereby to exert on the product a pressure of 60 g/cm$^2$ over the liquid acquisition zone thereof.
(c) Keep the weight on the product for 15 seconds.
(d) Remove the weight.
(e) Ascertain whether any liquid has been absorbed by the blotting paper.
(f) Repeat operations (b) to (e) nine times with a 15 second pause after each cycle.

Flexibility

This is measured by the test laid down in ASTM Standard D 1388-64 for measuring the stiffness of fabrics. For full details reference should be made to that Standard, which is incorporated herein by reference. In brief, however, what is done is to cause a strip of the material being tested to project over an edge of a platform, until the tip of the test specimen is depressed under its own wight to the point where the line joining the tip to the edge of the platform makes an angle with the horizontal of 41.5°. The length of the projecting portion is taken as the overhang length O, and the bending length c is calculated as 0/2. The flexural rigidity G is then calculated from this as:

$$G = Wxc^3 \text{ mg.cm}$$

This formula assumes that the weight is W in mg/cm$^2$.

Thickness

The thickness of the pantiliners is measured under a pressure of 20 g/cm$^2$ (0.2845 psi).

Synthetic urine

The composition was as given earlier in this description.
Details will now be given of a number of embodiments of a pantiliner according to the invention. In so doing, the nomenclature of Table 1 above will be used to identify the various layers of the products. It will, however, be noted that in Product 10 there are two adjacent absorbent core layers, of the same materials as one another but in different basis weights, which together give the desired total basis weight (120 g/m$^2$) for the core.
All the embodiments were hourglass in shape and had a length of 153 mm, a maximum width of 67 mm and a minimum width of 51 mm with all layers being of the same size and shape, except in the case of Product 10. The structures of Product 2 is shown in exploded isometric view in Figure 1 of the accompanying drawings. In the case of Product 10, the Absorbent Core (1) (denoted as 3') was smaller in plan than the pad, having a length of 143 mm, a maximum width of 57 mm and a minimum width of 41 mm, but Absorbent Core (2) (denoted as 3") was the same size as the pad. The construction of Product 10 is shown in Figure 2a, which is a plan view, and Figure 2b, which is a cross-section taken on line A-A in Figure 2a. In this construction, both absorbent corers are hourglass in shape. A layer of continuous adhesive 2" bonds the two together, and may also be sufficient to bond the composite core to the topsheet, though an optional discontinuous thin adhesive layer 2' may be provided between Absorbent Core (1) and the topsheet. Absorbent Core (2) is bonded to the backsheet by lines of adhesive.

| Product 1 | |
|---|---|
| Topsheet | None |
| Topsheet/core adhesive | None |
| Absorbent core | Hydrophilic, spunlaced, nonwoven material; 50g/m$^2$, 70% rayon, 30% PET (stretchable) |
| Core/backsheet adhesive | Hot-melt, elastomeric glue[1], 20 g/m$^2$ (elastic) |
| Backsheet | Hydrophobic, spunlaced, nonwoven material; 30g/m$^2$, 100% PET (stretchable) |
| PFA | Holt-melt Savarè LA 203, 24 g/m$^2$ |
| Footnote (1): holt-melt elastomeric adhesive described in detail above. | |

As will be seen, Product 1 has no topsheet, and accordingly the term "absorbent core" is to be understood in this context as meaning simply an absorbent member, and is not to be understood as carrying an implication that the member concerned is sandwiched between other members. Despite the absence of a topsheet, the product appears to be acceptable to users, in view of the relatively low volume of fluid which the product is to handle. The absence of a topsheet has the result, which can be seen from the results which are set out below, of making the product highly flexible.

| Product 2 | |
|---|---|
| Topsheet | Ring-rolled, apertured or perforated film (stretchable) |
| Topsheet/core adhesive | Hot-Melt glue lines, Savare PM 17, 0.06 g/liner m. (stretchable) |
| Absorbent core | Hydrophilic, spunlaced, nonwoven material; 50 g/m$^2$, 70% Rayon 30% PET (stretchable) |
| Core/backsheet adhesive | Hot-melt, elastomeric adhesive (1); 20 g/m$^2$ (elastic) |
| Backsheet | Hydrophobic, spunlaced, nonwoven material; 30 g/m$^2$, 100% PET (stretchable) |
| PFA | Hot-Melt Savarè LA203, 24 g/m$^2$ |

| Product 3 | |
|---|---|
| Topsheet | Hydrophilic Elastic Perforated Coverstock, 62 g/m$^2$ (2) (elastic) |
| Topsheet/core adhesive | Hot-Melt coated glue, Savarè PM 17, 5 g/m$^2$ (stretchable) |
| Absorbent Core | Creped tissue, 47 g/m$^2$ (stretchable) |
| Core/backsheet adhesive | Hot-Melt, coated glue, Savarè PM 17, 20 g/m$^2$ (stretchable) |
| Backsheet | Elastic film, EXX 500 38 g/m$^2$ (3) (elastic) |
| PFA | Hot-Melt Savarè LA203, 24 g/m$^2$ |
| Footnote (2): The material used was as described above with reference to Figures 10 to 17.<br>Footnote (3): EXX500 is available from Exxon Corporation. | |

| Product 4 | |
|---|---|
| Topsheet | Hydrophilic Elastic Perforated Coverstock, 62 g/m$^2$ (2) (elastic) |
| Topsheet/core adhesive | Hot-melt coated glue, Savarè PM 17, 5 g/m$^2$ (stretchable) |
| Absorbent Core | Hydrophilic, spunlaced, nonwoven material, 50 g/m$^2$, 70% Rayon 30% PET (stretchable) |
| Core/backsheet adhesive | Hot-melt, elastomeric adhesive (1), 20 g/m$^2$ (elastic) |
| Backsheet | Hydrophobic, spunlaced, nonwoven material; 40 g/m$^2$, 100% PET (stretchable) |
| PFA | Hot-melt Savarè LA203, 24 g/m$^2$ |

| Product 5 | |
|---|---|
| Topsheet | Hydrophilic Elastic Perforated Coverstock, 62 g/m$^2$ (2) (elastic) |
| Topsheet/core adhesive | Hot-melt coated glue, Savarè PM 17, 5 g/m$^2$ (stretchable) |
| Absorbent Core | Hydrophilic, spunlaced, nonwoven material, 50 g/m$^2$, 70% Rayon 30% PP (polypropylene) (stretchable) |
| Core/backsheet adhesive | Hot-melt coated glue, Savarè PM 17, 20 g/m$^2$ (stretchable) |
| Backsheet | Elastic film, EXX 500 38 g/m$^2$ (3) (elastic) |
| PFA | Hot-melt Savarè LA203, 24 g/m$^2$ |

| Product 6 | |
|---|---|
| Topsheet | Hydrophilic, spunlaced, nonwoven material, 30 g/m$^2$, 70% Rayon 30% PET (stretchable) |
| Topsheet/core adhesive | Hot-melt coated glue, Savarè PM 17, 5 g/m$^2$ (stretchable) |
| Absorbent Core | Creped tissue, 47 g/m$^2$ (stretchable) |
| Core/backsheet adhesive | Hot-melt, elastomeric adhesive (1), 20 g/m$^2$ (elastic) |
| Backsheet | Hydrophobic, spunlaced, nonwoven material, 40 g/m$^2$, PET (stretchable) |
| PFA | Hot-melt Savarè LA203, 24 g/m$^2$ |

| Product 7 | |
|---|---|
| Topsheet | Ring-rolled, apertured or perforated film (stretchable) |
| Topsheet/core adhesive | Hot-melt coated glue, Savarè PM 17, 10 g/m$^2$ (stretchable) |
| Absorbent Core | Hydrophilic, spunlaced, nonwoven material, 70 g/m$^2$, 70% Rayon 30% PP (stretchable) |
| Core/backsheet adhesive | Hot-melt, elastomeric adhesive (1), 20 g/m$^2$ (elastic) |
| Backsheet | Hydrophobic, spunlaced, nonwoven material, 40 g/m$^2$, 100% PP (stretchable) |
| PFA | Hot-melt Savarè LA203, 24 g/m$^2$ |

| Product 8 | |
|---|---|
| Topsheet | Hydrophilic Elastic Perforated Coverstock, 62 g/m$^2$ (2) (elastic) |
| Topsheet/core adhesive | Hot-melt coated glue, Savare PM 17, 10g/m$^2$ (stretchable) |
| Absorbent Core | Hydrophilic, spunlaced, nonwoven material, 70g/m$^2$, 70% Rayon 30% PP (stretchable) |
| Core/backsheet adhesive | Hot-melt, elastomeric adhesive (1), 20g/m$^2$ (elastic) |
| Bacxsheet | Hydrophobic, spunlaced, nonwoven material, 40g/m$^2$, 100% PP (stretchable) |
| PFA | Hot-melt Savarè LA203, 24g/m$^2$ |

| Product 9 | |
|---|---|
| Topsheet | None |
| Topsheet/core adhesive | None |
| Absorbent Core | Hydrophilic, spunlaced, nonwoven material, 70g/m$^2$, 70% Rayon 30% PP (stretchable) |
| Core/backsheet adhesive | Hot-melt, elastomeric adhesive (1), 20g/m$^2$ (elastic) |
| Backsheet | Hydrophobic, spunlaced, nonwoven material, 40g/m$^2$, 100% PP (stretchable) |
| PFA | Hot-melt Savare LA203, 24g/m$^2$ |

Like Product 1, this has no topsheet, and the same comments apply as regards this fact as are set our above in relation to Product 1.

| Product 10 | |
|---|---|
| Topsheet | Ring-rolled, apertured or perforated film (stretchable) |
| Topsheet/core adhesive | Hot-melt coated glue, Savarè PM 17, 10g/m$^2$ (stretchable) |
| Absorbent Core (1) | Hydrophilic, spunlaced, nonwoven material, 70g/m$^2$, 70% Rayon 30% PP (stretchable) |
| Absorbent Core (2) | Hydrophilic, spunlaced, nonwoven material, 50g/m$^2$, 70% Rayon 30% PET (stretchable) |
| Core/backsheet adhesive | Hot-melt, elastomeric adhesive (1); 20g/m$^2$ (elastic) |
| Backsheet | Hydrophobic, spunlaced, nonwoven material, 40g/m$^2$, 100% PP (stretchable) |
| PFA | Hot-melt Savarè LA203, 24g/m$^2$ |

<u>Load and pad set tests</u>

The elastic behaviour of some of the embodiments described above (Products 1, 2, 4, 7, 8 and 9), a conventional pantiliner sold by Johnson & Johnson as "Carefree Normal", and a cotton panty in which the product of the invention might be used, were investigated in a test described below. In this test, a sample of each product was subjected to three hysteresis cycles by means of a tensile tester designed to apply to each sample a load which, in each cycle, gradually increases and then gradually decreases.

Each product is first conditioned, by keeping it for at least 12 hours in a room at a temperature of $23 \pm 2°$ C and a relative humidity of $50 \pm 2\%$. A strip one inch (2.54 cm) wide was then cut from the product the length of the strip being either parallel to the length of the product (if it is desired to measure MD elastic behaviour) or perpendicular to the length of the product (if it is desired to measure CD elastic behaviour). In the test of which the results are given below it was CD elastic behaviour which was measured.

The sample is laid unrestrained on a table. The length of the part of the sample which will be tested is marked on the sample itself. The distance between the clamps of the tensile tester is set to the same length that has been previously marked on the specimen. Each sample is clamped in the tensile tester in such a way as to enable the tester to apply a load lengthwise of the strip and to avoid slacks or tensions. The load cell must record increase as soon as the test starts. The following steps were then carried out:

1. Increase the load gradually from zero up to a value at which the length of that part of the sample which is under load is 125% of its initial length, continuously recording the load and corresponding length as this is being done.
2. Decrease the load gradually until the distance between the clamp returns to its starting point, continuously recording the load and corresponding length as this is being done.
3. Hold the sample for 30 seconds.
4. Repeat steps 1 to 3 two further times.

The results obtained are set out in Table 2 below, except for the Carefree Normal sample, which had too little elasticity for it to be capable of undergoing the test, for each of the three hysteresis cycles. In each case, the table records the peak load, i.e. the load in g/inch required to achieve 25% elongation, the percentage set, i.e. the percentage amount by which the length of the sample exceeded its original length at the end of the each cycle at a load of 0.05 N/inch. A value of 0.05 N, rather than zero is chosen, to avoid possible reading problems with the computer software via which measurements are made. The table also records the energy dissipated in the course of the cycle. This last value, measured in $10^{-3}$J, is determined from the area within the hysteresis cycle.

Some of the results of Table 2, together with other properties of the samples, are set out in Table 3.

## TABLE 2

|  |  | PEAK LOAD | % SET | ENERGY $(10^{-3}$ J) |
|---|---|---|---|---|
| Cotton panty 100% cotton | CYCLE I | 127 | 5% | 3.15 |
|  | CYCLE II | 123 | 6% | 2.38 |
|  | CYCLE III | 122 | 6% | 2.35 |
| product # 1 | CYCLE I | 122 | 10% | 5.10 |
|  | CYCLE II | 111 | 11% | 2.54 |
|  | CYCLE III | 98 | 11% | 2.50 |
| product # 2 | CYCLE I | 140 | 10% | 5.28 |
|  | CYCLE II | 126 | 10% | 2.73 |
|  | CYCLE III | 111 | 11% | 1.88 |
| product # 4 | CYCLE I | 349 | 6% | 13.7 |
|  | CYCLE II | 319 | 6% | 9.67 |
|  | CYCLE III | 303 | 7% | 8.14 |
| product # 7 | CYCLE I | 117 | 11% | 4.77 |
|  | CYCLE II | 103 | 11% | 2.91 |
|  | CYCLE III | 99 | 12% | 2.67 |
| product # 8 | CYCLE I | 218 | 6% | 7.90 |
|  | CYCLE II | 214 | 7% | 4.19 |
|  | CYCLE III | 203 | 7% | 4.17 |
| product # 9 | CYCLE I | 102 | 11% | 3.85 |
|  | CYCLE II | 93 | 12% | 2.44 |
|  | CYCLE III | 86 | 12% | 1.79 |

TABLE 3

| Product | 1 | 2 | 4 | 7 | 8 | 9 | Carefree Normal | Cotton Panty |
|---|---|---|---|---|---|---|---|---|
| Stretch (N/inch) (Peak load/1st cycle) | 122 | 140 | 349 | 117 | 218 | 102 | not noticeable | 127 |
| Pad set (%) (1st cycle/3rd cycle) | 10/11 | 10/11 | 6/7 | 11/12 | 6/7 | 11/12 | not noticeable | 5/6 |
| Wet through | None | None | None | None | None | None | None | Yes |
| Thickness (mm) | 0.83 | 1.21 | 1.13 | 1.28 | 1.36 | 0.97 | 3.10 | 0.40 |
| Flexibility (mg.cm) | 500 | 1230 | 2720 | 4000 | n.a. | 1867 | >7770 | n.a. |

n.a. stands for "not available"

A number of points emerge from Table 2 and 3. Firstly, it will be seen that all the Products tested had a low value of pad set, (not more than 12% after three cycles, even in the worst case, and 6% in the best case), indicating that the Products will not lose their elasticity in use. Secondly, Products 1, 2, 7 and 9 are seen to be particularly advantageous in that the loads required to reach 25% are low, and are comparable to those required in the case of the cotton panty. Consequently, during use the pantiliner and panty will stretch to substantially the same extent as one another in response to any given force, and the pantiliner will therefore behave, for practical purposes, as though it were part of the panty itself.

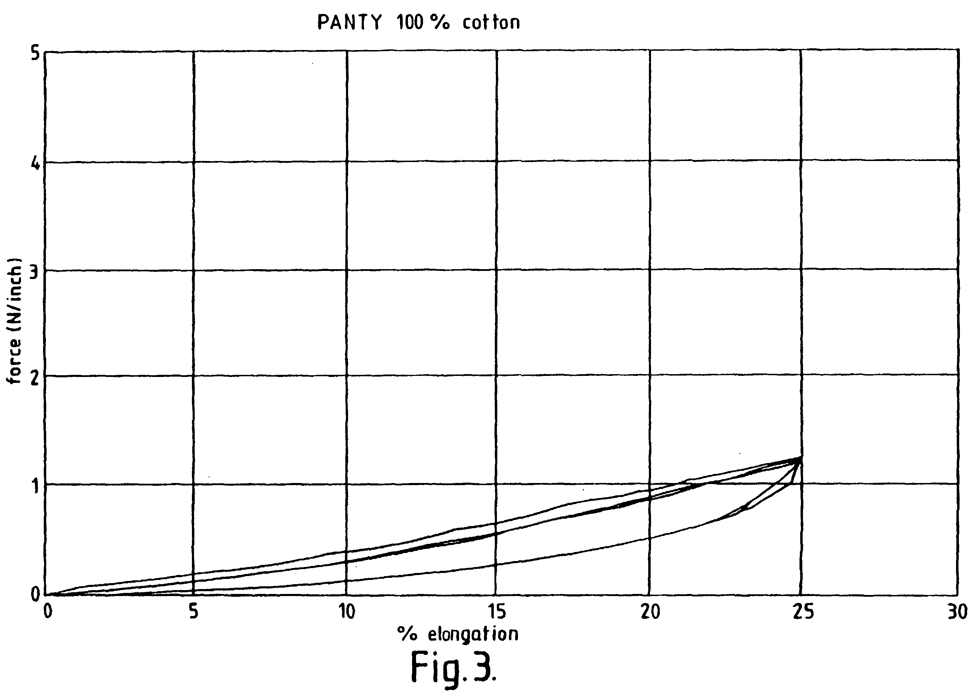

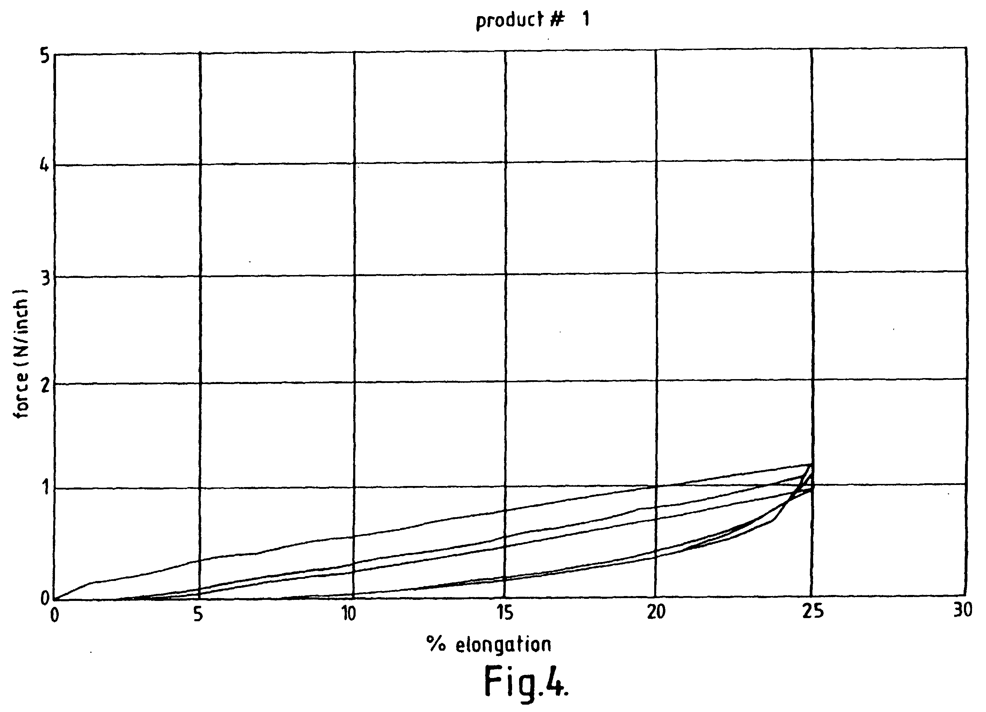

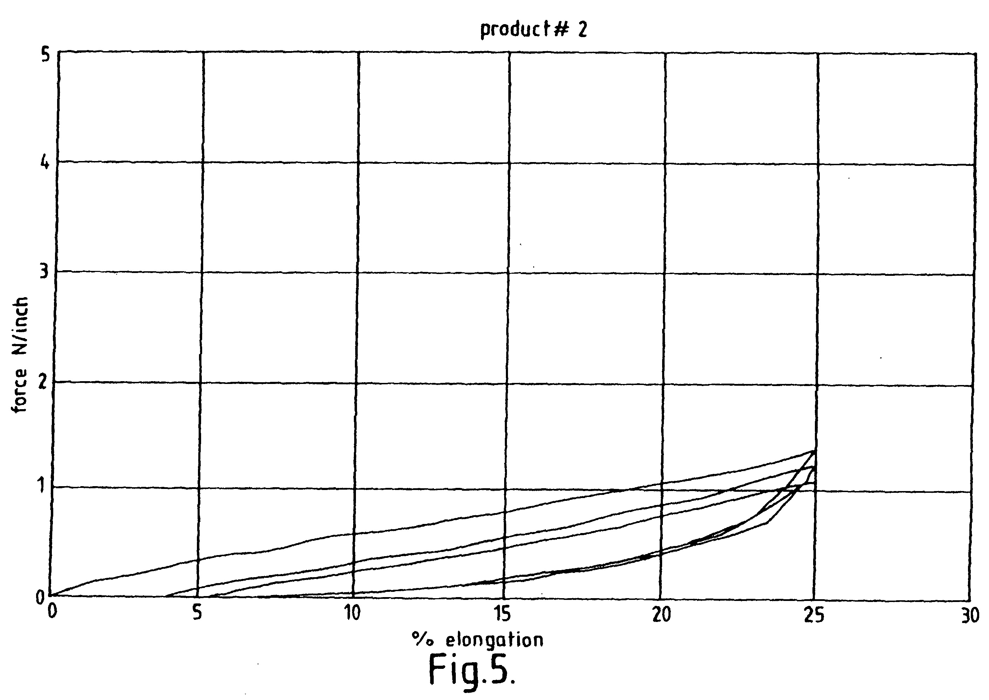

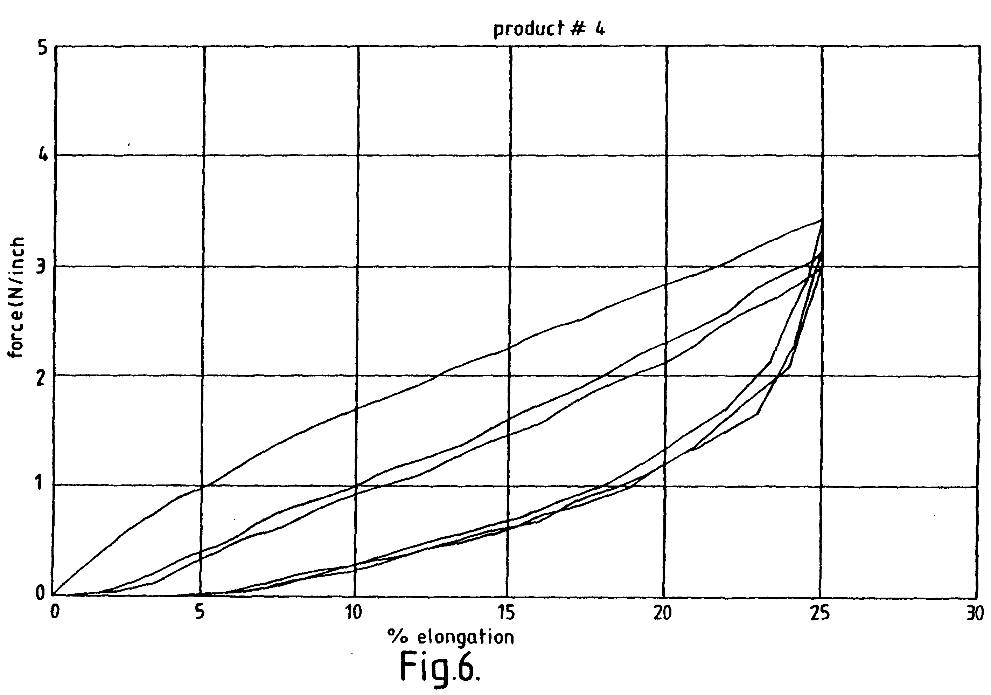

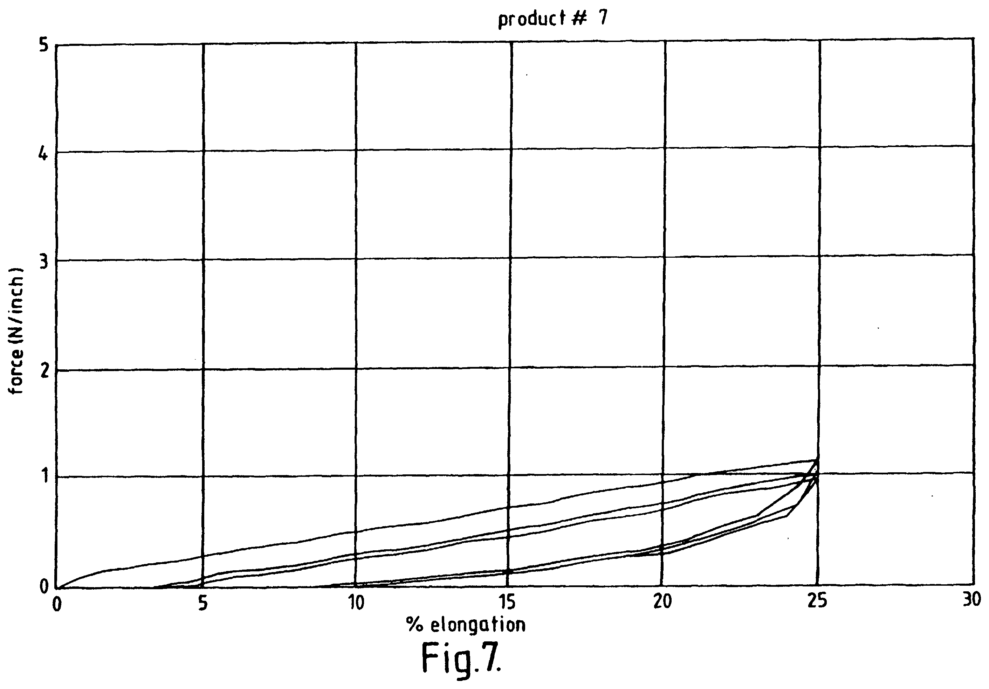

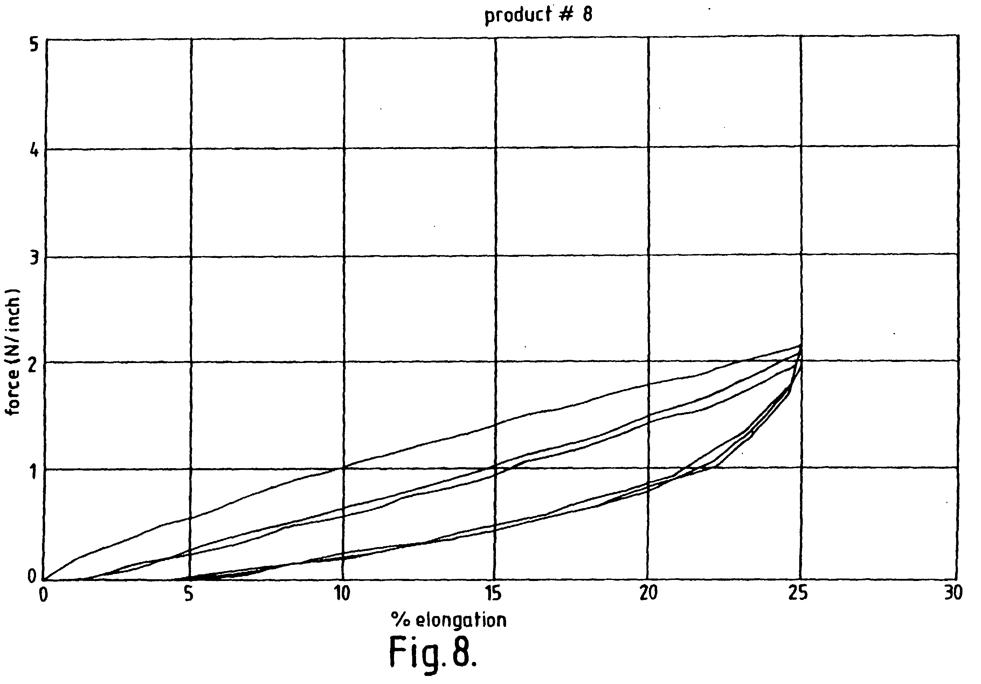

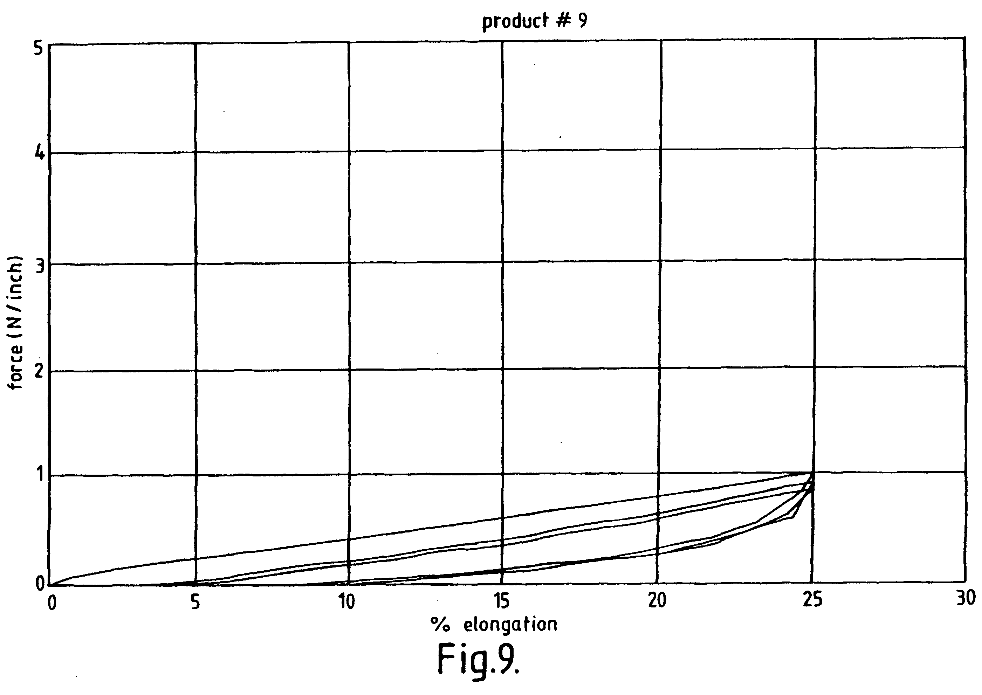

Figures 3 to 9 are hysteresis cycles which show the elastic behaviour of the products referred to in Table 2. As can be seen, the amount of hysteresis is not great in the case of any of those according to the invention, indicating that the products will retain their elastic properties to a substantial extent during repeated stretching and relaxation. Figure 3 is a hysteresis cycle for the cotton panty. This can be seen to be broadly similar to Figures 4 to 9. There is no such cycle for the comparative article "Carefree Normal", since this is almost completely inelastic.

**Claims**

1. A multi-layer, fluid-absorbent sanitary article for use with a panty garment, the article having means of attachment (6) to the interior of the garment in the crotch area thereof, **characterized in that** the article is flexible, and elastic in at least one direction, the elasticity being such that in said at least one direction the loading force required to extend a sample of the material 1 inch (2.54 cm) in width to 125% of its original length is not more than 400g, and the residual stretch (pad set) after the sample has been stretched three times to 125% of its original length and relaxed after each stretch is not more than 15% of its original length.

2. An article according to claim 1, wherein the said loading force is not more than 250g.

3. An article according to claim 2, wherein the said loading force is not more than 150g.

4. An article according to claim 3, wherein the said loading force is not more than 100g.

5. An article according to any preceding claim, wherein the said loading force is at least 60g.

6. An article according to any preceding claim, wherein said pad set is not more than 12%.

7. An article according to claim 6, wherein said pad set is not more than 8%.

8. An article according to any preceding claim, having a thickness of less than 2mm as measured under a pressure of (20g/cm$^2$) 1961Pa.

9. An article according to claim 8, wherein the thickness under the said pressure is in the range of from 0.5 to 1.6mm.

10. An article according to any preceding claim, which has a flexibility, as measured according to ASTM Standard D 1388-64, is not more than 5000 mg.cm.

11. An article according to claim 10, wherein the flexibility is not more than 3000 mg.cm.

12. An article according to claim 10 or 11, wherein the flexibility is at least 500 mg.cm.

13. An article according to any preceding claim, wherein the article is elongate and has a direction of elasticity transverse to the length thereof.

14. An article according to any one of claims 1 to 12, wherein the article is elongate and has a direction of elasticity parallel to the length thereof.

15. An article according to any one of claims 1 to 12, wherein the article is elongate and has directions of elasticity both transverse and parallel to the length thereof.

16. An article according to any one of claims 13 to 15, wherein the article has an hourglass shape.

17. An article according to any preceding claim, wherein the layers are substantially identical to one another in shape and size, and are substantially in alignment with one another.

18. An article according to any preceding claim, which comprises a layer of absorbent material (3) exposed to the exterior on one face thereof, and joined in face-to-face relationship on its opposite face, by means of an adhesive connection, with a backsheet (5) of water-impermeable material.

19. An article according to any one of claims 1 to 17, which comprises a layer of absorbent material (3) sandwiched between a topsheet (1) of water-permeable material and a backsheet (5) of water-impermeable material, the absorbent material (3) being joined to the backsheet (5) and topsheet (1) by adhesive connections (2).

20. An article according to claim 18 or 19, wherein at least the absorbent material (3) is elastic, and each of the other layers is elastic or stretchable but inelastic.

21. An article according to claim 18, 19 or 20, wherein at least the backsheet (5) is elastic, and each of the other layers is elastic or stretchable but inelastic.

22. An article according to any one of claims 19 to 21, wherein at least the topsheet (1), where provided, is elastic, and each of the other layers is elastic or stretchable but inelastic.

23. An article according to claim 22, wherein the topsheet (1) is a structure having perforations (30) which extend therethrough, the structure comprising:

   (a) an upper layer (110) intended to face outwardly of the absorbent body and comprising a non-woven fibrous material;
   (b) an intermediate layer (111) comprising an elastic film; and
   (c) a lower layer (112) intended to face inwardly towards the absorbent body and comprising a non-woven fibrous material; the upper and lower layers (110, 112) being connected to the intermediate layer (111) substantially only around the perimeters of the perforations.

24. An article according to any one of claims 18 to 23, wherein at least the adhesive connection between the absorbent material and the backsheet (5) is elastic, and each of the other layers is elastic or stretchable but inelastic.

25. An article according to any one of claims 19 to 24, wherein at least the adhesive connection (2) between the absorbent material (3) and the topsheet (1), where provided, is elastic, and each of the other layers is elastic or stretchable but inelastic.

26. An article according to any one of claims 18 to 25, wherein at least one of the topsheet (1) (where provided), absorbent material (3), backsheet (5), and adhesive connections(s) (2), is stretchable but inelastic.

27. An article according to any one of claims 18, 19 and 21 to 26, wherein the absorbent material (3) is stretchable but inelastic and is selected from the group consisting of spunlaced materials, creped materials, and slitted materials.

28. An article according to any one of claims 18 to 21 and 23 to 27, wherein the topsheet (1), where provided, is stretchable but inelastic and is a ring-rolled material.

29. An article according to any one of claims 18 to 20 and 22 to 28, wherein the backsheet (5) is stretchable but inelastic and is formed of a spunlaced material.

30. An article according to any one of claims 18 to 28, wherein the backsheet (5) is elastic and is formed of an elastomeric film.

31. An article according to any one of claims 18 to 30, wherein the adhesive connection(s) (2) is provided by an elastomeric hot melt adhesive composition comprising at least one thermoplastic elastomer and at least one tackifying resin, the thermoplastic elastomer(s) being a styrene/butadiene/styrene (SBS) copolymer or a blend of styrene/butadiene/styrene with styrene/isoprene/styrene (SIS) in which SIS is present in an amount equal to or less than 50% by weight of the total block copolymer, the composition being **characterised in that**:

   (a) it is capable of bonding, when applied from the molten state, plastic and/or cellulosic materials with a 90° peel force not lower than 0.5 N/cm,
   (b) it has a tensile strength retention after 50 cycles (as herein defined) of at least 40%;
   (c) it has a viscosity of 120,000 cps or less at 180°C and an applied shear of 80 sec$^{-1}$.

**32.** An article according to any one of claims 1 to 17, wherein at least one of the layers is elastic and each of the other layers is elastic or stretchable but inelastic.

**33.** An elastic, fluid-absorbent sanitary article according to claim 1 for use with a panty garment, the article having means of attachment (6) to the interior of the garment in the crotch area thereof, the article comprising a plurality of layers joined in face-to-face relationship with one another by means of at least one adhesive connection, the said adhesive connection or one of said adhesive connections, being provided by an elastic adhesive, whereby to provide, at least in part, the elasticity of the article.

**34.** An article according to claim 33, which comprises a layer of absorbent material (3) exposed to the exterior on one face thereof, and joined in face-to-face relationship on its opposite face, by means of an adhesive connection provided by an elastic adhesive, with a backsheet (5) of water-impermeable material.

**35.** An article according to claim 33, which comprises a layer of absorbent material (3) sandwiched between a topsheet (1) of water permeable material and a backsheet (5) of water-impermeable material, the absorbent material (3) being joined to the backsheet (5) and topsheet (1) by adhesive connections at least one of which is provided by an elastic adhesive.

**36.** An article according to any one of claims 33 to 35, wherein the adhesive is an elastomeric hot melt adhesive.

**37.** A fluid-absorbent article according to claim 1 for use with a panty garment, which comprises a layer of absorbent material exposed to the exterior on one face thereof, and joined in face-to-face relationship on its opposite face, by means of an adhesive connection, with a backsheet of water-impermeable material.

**38.** An article according to claim 37, having a thickness of less than 2mm.

**39.** An article according to claim 37, having a thickness of less than 1mm.

**40.** A fluid-absorbing sanitary article according to any preceding claim, wherein the layers thereof, and the connections therebetween, are such as to render it permeable to water vapour and other gases.

**41.** A fluid-absorbent sanitary article according to claim 1 for use with a cotton panty garment, the article having means (6) for removably attaching it to the interior of the panty garment in the crotch area thereof, wherein the stress-strain characteristics of the said article correspond to a substantial extent to those of the said crotch area.

**42.** An absorbent article for according to claim 1 adhesive attachment to an undergarment, wherein adhesive is applied to a surface of the article, said surface being outwardly defined by a perimeter, the adhesive being applied only to at least one portion of the said surface at or adjacent the said perimeter, the remainder of the surface being adhesive-free.

**43.** An article according to claim 42, wherein at least 25% of the said surface is adhesive-free.

**44.** An article according to claim 43, wherein at least 50% of the said surface is adhesive-free.

**45.** An article according to any one of claims 42 to 44, wherein the said portion consists of a strip extending around the said surface adjacent the said perimeter.

**46.** An article according to claim 45, wherein the said strip extends completely to the said perimeter.

**47.** An article according to claim 45, wherein the said strip is separated from the said perimeter by a narrow adhesive-free region over the complete length thereof.

**48.** An article according to claim 45, wherein the said strip is separated from the said perimeter by a narrow adhesive-free region except over a minor part thereof.

**49.** An article according to claim 45, wherein the said strip extends completely to the said perimeter except over a minor part thereof.

**50.** An article according to any one of claims 42 to 44, wherein the adhesive is applied to a pair of said portions, said portions being separated from one another by an adhesive-free region.

**51.** An article according to claim 50, which is elongate, and wherein the pair of said portions are themselves elongate and extend along laterally opposite edges of the articles, the adhesive-free region being also elongate.

**52.** An article according to any one of claims 45 to 49, which is in the form of a pantiliner and in which the said strip is 4 to 10mm wide.

**53.** An article according to claim 52, wherein the said strip is about 5mm wide.

**54.** An article according to claim 52 or 53, as dependent on claim 6 or 7, wherein the said narrow adhesive-free region is not more that 3mm wide.

**55.** An article according to claim 54, wherein the said narrow adhesive-free region is from 1 to 2mm wide.

**56.** An article according to claim 52 or 53, as dependent on claim 45, wherein said minor part is provided by a plurality of adhesive-free regions spaced apart from one another and each not more than about 2mm wide.

**57.** An article according to claim 51, which is in the form of a pantiliner and in which the elongate adhesive-free region is from 15 to 43mm wide.

**58.** An article according to any one of claims 42 to 57, which is elastically stretchable.

**59.** An article according to any one of claims 42 to 47, which is non-elastically stretchable.

**60.** An article according to any one of claims 42 to 57, which is non-stretchable.

**61.** An article according to any one of claims 42 to 60, wherein the said adhesive is based on a styrene-ethylene-butylene-styrene block copolymer.

**62.** A stretchable, absorbent article according to claim 1 for adhesive attachment to an undergarment, wherein a stretchable adhesive is applied to a surface of the article, said surface being outwardly defined by a perimeter, the adhesive being applied to a major portion of the said surface, and extending at least substantially to the said perimeter over a major portion of the said perimeter.

**63.** An article according to claim 62, wherein the adhesive extends completely to the said perimeter over a major portion of said perimeter.

**64.** An article according to claim 63, wherein the said surface has at least one adhesive-free region.

**65.** An article according to claim 64, which is elongate and which has an adhesive-free region at each end thereof.

**66.** An article according to claim 64, which is hourglass in shape and has an adhesive-free region at each corner thereof.

**67.** An article according to claim 62, wherein the said surface has an adhesive-free region in the form of a narrow adhesive-free strip extending along at least a substantial part of the said perimeter.

**68.** An article according to claim 67, wherein the said strip extends completely along the said perimeter.

**69.** An article according to any one of claims 62 to 68, which is in the form of a pantiliner.

**70.** An article according to claim 65, which is in the form of a pantiliner and in which each of the said adhesive-free regions extends a distance of from 2 to 4mm from the adjacent end of the pantiliner.

**71.** An article according to claim 70, wherein the said distance is about 3mm.

**72.** An article according to claim 66, which is in the form of a pantiliner and in which each of the said adhesive-free regions extends a distance of from 2 to 4mm from the adjacent lateral edge of the pantiliner.

73. An article according to claim 72, wherein the said distance is about 3mm.

74. An article according to claim 67 or 68, which is in the form of a pantiliner and in which the said adhesive-free strip has a width of less than 3mm.

75. An article according to claim 74, wherein the said width is from 1 to 2mm.

76. An article according to any one of claims 62 to 75, wherein the said stretchable adhesive is based on a styrene-ethylene-butylene-styrene block copolymer.

77. An article according to any one of claims 62 to 76, which is elastically stretchable.

**Patentansprüche**

1. Mehrlagiger, fluidabsorbierender Hygieneartikel zur Verwendung mit einem Slip-Kleidungsstück, wobei der Artikel ein Mittel zur Befestigung (6) an der Innenseite des Kleidungsstückes in dessen Schrittbereich aufweist, **dadurch gekennzeichnet, dass** der Artikel flexibel und mindestens in einer Richtung elastisch ist, wobei die Elastizität derart ist, dass in der mindestens einen Richtung die Beanspruchungskraft, die erforderlich ist, um eine Probe des 1 Inch (2,54 cm) breiten Materials auf 125 % ihrer ursprünglichen Länge auszudehnen, nicht größer als 400 g ist, und die verbleibende Dehnung (Pad-Verformungsrest), nachdem die Probe dreimal auf 125 % ihrer ursprünglichen Länge gedehnt worden ist und nach jeder Dehnung entspannt wurde, nicht größer als 15 % ihrer ursprünglichen Länge ist.

2. Artikel nach Anspruch 1, wobei die Beanspruchungskraft nicht größer als 250 g ist.

3. Artikel nach Anspruch 2, wobei die Beanspruchungskraft nicht größer als 150 g ist.

4. Artikel nach Anspruch 3, wobei die Beanspruchungskraft nicht größer als 100 g ist.

5. Artikel nach einem vorherigen Anspruch, wobei die Beanspruchungskraft mindestens 60 g ist.

6. Artikel nach einem vorherigen Anspruch, wobei der Pad-Verformungsrest nicht größer als 12 % ist.

7. Artikel nach Anspruch 6, wobei der Pad-Verformungsrest nicht größer als 8 % ist.

8. Artikel nach einem vorherigen Anspruch, der eine Dicke von weniger als 2 mm, gemessen unter einem Druck von 1961 Pa (20g/cm$^2$), aufweist.

9. Artikel nach Anspruch 8, wobei die Dicke unter dem genannten Druck im Bereich von 0,5 bis 1,6 mm liegt.

10. Artikel nach einem vorherigen Anspruch, der eine Flexibilität, gemessen gemäß dem ASTM Standard D 1388-64, von nicht größer als 5000 mg.cm aufweist.

11. Artikel nach Anspruch 10, wobei die Flexibilität nicht größer als 3000 mg.cm ist.

12. Artikel nach Anspruch 10 oder 11, wobei die Flexibilität mindestens 500 mg.cm ist.

13. Artikel nach einem vorherigen Anspruch, wobei der Artikel länglich ist und eine zu seiner Länge transversale Elastizitäts-Richtung aufweist.

14. Artikel nach einem der Ansprüche 1 bis 12, wobei der Artikel länglich ist und eine zu seiner Länge parallele Elastizitäts-Richtung aufweist.

15. Artikel nach einem der Ansprüche 1 bis 12, wobei der Artikel länglich ist und zu seiner Länge sowohl transversale als auch parallele Elastizitäts-Richtungen aufweist.

**16.** Artikel nach einem der Ansprüche 13 bis 15, wobei der Artikel eine Sanduhr-Form aufweist.

**17.** Artikel nach einem vorherigen Anspruch, wobei die Lagen gegenseitig im Wesentlichen identisch in Form und Größe sind und im Wesentlichen zueinander ausgerichtet sind.

**18.** Artikel nach einem vorherigen Anspruch, welcher eine Lage eines absorbierenden Materials (3) aufweist, die auf ihrer einen Seite der Außenseite zugewandt offen liegt und die in Seite-an-Seite-Beziehung auf ihrer abgewandten Fläche durch eine Klebstoff-Verbindung mit einer Außenlage (5) eines wasserundurchlässigen Materials verbunden ist.

**19.** Artikel nach einem der Ansprüche 1 bis 17, welcher eine Lage eines absorbierenden Materials (3) aufweist, die zwischen einer Decklage (1) eines wasserdurchlässigen Materials und einer Außenlage (5) eines wasserundurchlässigen Materials angeordnet ist, wobei das absorbierende Material (3) mit der Außenlage (5) und der Decklage (1) durch Klebstoff-Verbindungen (2) verbunden ist.

**20.** Artikel nach Anspruch 18 oder 19, wobei mindestens das absorbierende Material (3) elastisch ist und jede der anderen Lagen elastisch oder dehnbar, aber unelastisch ist.

**21.** Artikel nach Anspruch 18, 19 oder 20, wobei mindestens die Außenlage (5) elastisch ist und jede der anderen Lagen elastisch oder dehnbar, aber unelastisch ist.

**22.** Artikel nach einem der Ansprüche 19 bis 21, wobei mindestens die Decklage (1), wo vorgesehen, elastisch ist und jede der anderen Lagen elastisch oder dehnbar, aber unelastisch ist.

**23.** Artikel nach Anspruch 22, wobei die Decklage (1) eine Struktur mit Perforationen (30) ist, die sich durch diese erstrecken, wobei die Struktur umfasst:

(a) eine Oberschicht (110), die vorgesehen ist, um nach außen von dem absorbierenden Körper gewandt zu sein, und ein faseriges Nonwoven-Material umfasst;
(b) eine Zwischenschicht (111) mit einer elastischen Folie; und
(c) eine Unterschicht (112), die vorgesehen ist, um nach innen in Richtung auf den absorbierenden Körper gewandt zu sein, und ein faseriges Nonwoven-Material aufweist; wobei die Ober- und Unterschichten (110, 112) mit der Zwischenschicht (111) im Wesentlichen nur um die äußeren Begrenzungen der Perforationen verbunden sind.

**24.** Artikel nach einem der Ansprüche 18 bis 23, wobei mindestens die Klebstoff-Verbindung zwischen dem absorbierenden Material und der Außenlage (5) elastisch ist und jede der anderen Lagen elastisch oder dehnbar, aber unelastisch ist.

**25.** Artikel nach einem der Ansprüche 19 bis 24, wobei mindestens die Klebstoff-Verbindung (2) zwischen dem absorbierenden Material (3) und der Decklage (1), wo vorgesehen, elastisch ist und jede der anderen Lagen elastisch oder dehnbar, aber unelastisch ist.

**26.** Artikel nach einem der Ansprüche 18 bis 25, wobei die Decklage (1) (wo vorgesehen), das absorbierende Material (3), die Außenlage (5) und der(die) Klebstoff-Verbindung(en) (2) dehnbar, aber unelastisch ist(sind).

**27.** Artikel nach einem der Ansprüche 18, 19 und 21 bis 26, wobei das absorbierende Material (3) dehnbar, aber unelastisch ist und gewählt ist aus der Gruppe bestehend aus Spunlaced-Materialien, gekreppten Materialien und geschlitzten Materialien.

**28.** Artikel nach einem der Ansprüche 18 bis 21 und 23 bis 27, wobei die Decklage (1), wo vorgesehen, dehnbar, aber unelastisch ist und ein ring-gerolltes Material ist.

**29.** Artikel nach einem der Ansprüche 18 bis 20 und 22 bis 28, wobei die Außenlage (5) dehnbar, aber unelastisch ist und aus einem Spunlaced-Material gebildet ist.

**30.** Artikel nach einem der Ansprüche 18 bis 28, wobei die Außenlage (5) elastisch ist und aus einer Elastomer-Folie gebildet ist.

**31.** Artikel nach einem der Ansprüche 18 bis 30, wobei die Klebstoff-Verbindung(en) (2) durch eine elastomere Heiß-Schmelz-Klebstoff-Zusammensetzung gebildet ist(sind), die mindestens ein thermoplastisches Elastomer und mindestens ein klebrigmachendes Harz umfasst, wobei das(die) thermoplastische(n) Elastomer(e) ein Styrol/Butadien/Styrol (SBS) Copolymer oder ein Gemisch von Styrol/Butadien/Styrol mit Styrol/Isopren/Styrol (SIS) ist, in welchem SIS in einer Menge vorliegt, die gleich oder kleiner 50 Gew.-% des gesamten Block-Copolymers ist, wobei die Zusammensetzung **dadurch gekennzeichnet ist, dass**:

(a) diese in der Lage ist, wenn diese aus dem geschmolzenen Zustand aufgebracht wird, Kunststoff und/oder Zellulose-Materialien mit einer 90°-Schälkraft von nicht weniger als 0,5 N/cm zu binden,
(b) diese eine Zugkraft-Retention nach 50 Zyklen (wie hierin definiert) von mindestens 40 % aufweist;
(c) diese eine Viskosität von 120000 cps oder weniger bei 180°C und einer aufgebrachten Scherung von 80 $s^{-1}$ aufweist.

**32.** Artikel nach einem der Ansprüche 1 bis 17, wobei mindestens eine der Lagen elastisch ist und jede der anderen Lagen elastisch oder dehnbar, aber unelastisch ist.

**33.** Elastischer, fluidabsorbierender Hygieneartikel nach Anspruch 1 zur Verwendung mit einem Slip-Kleidungsstück, wobei der Artikel Befestigungsmittel (6) an der Innenseite des Kleidungsstückes in dessen Schrittbereich aufweist, wobei der Artikel eine Vielzahl von Lagen aufweist, die in gegenüberliegender Beziehung miteinander durch mindestens eine Klebstoff-Verbindung verbunden sind, wobei die Klebstoff-Verbindung oder eine der Klebstoff-Verbindungen durch einen elastischen Klebstoff gebildet ist, wodurch zumindest teilweise die Elastizität des Artikels bereitgestellt wird.

**34.** Artikel nach Anspruch 33, welcher eine Lage eines absorbierenden Materials (3) aufweist, die mit einer Seite der Außenseite offen zugewandt liegt und in Seite-an-Seite-Beziehung auf ihrer abgewandten Fläche durch eine Klebstoff-Verbindung, die durch einen elastischen Klebstoff gebildet ist, mit einer Außenlage (5) eines wasserundurchlässigen Materials verbunden ist.

**35.** Artikel nach Anspruch 33, welcher eine Lage eines absorbierenden Materials (3) umfasst, die zwischen einer Decklage (1) eines wasserdurchlässigen Materials und einer Außenlage (5) eines wasserundurchlässigen Materials angeordnet ist, wobei das absorbierende Material (3) mit der Außenlage (5) und der Decklage (1) durch Klebstoff-Verbindungen verbunden ist, wobei mindestens eine von diesen durch einen elastischen Klebstoff gebildet ist.

**36.** Artikel nach einem der Ansprüche 33 bis 35, wobei der Klebstoff ein elastomerer Heiß-Schmelz-Klebstoff ist.

**37.** Fluidabsorbierender Artikel nach Anspruch 1 zur Verwendung mit einem Slip-Kleidungsstück, welcher eine Lage eines absorbierenden Materials aufweist, die auf einer Seite der Außenseite offen zugewandt liegt und in Seite-an-Seite-Beziehung auf ihrer abgewandten Fläche durch eine Klebstoff-Verbindung mit einer Außenlage eines wasserundurchlässigen Materials verbunden ist.

**38.** Artikel nach Anspruch 37 mit einer Dicke von weniger als 2 mm.

**39.** Artikel nach Anspruch 37 mit einer Dicke von weniger als 1 mm.

**40.** Fluidabsorbierender Hygieneartikel nach einem vorherigen Anspruch, wobei dessen Lagen und die Verbindungen dazwischen derart sind, um diesen durchlässig für Wasserdampf und andere Gase zu machen.

**41.** Fluidabsorbierender Hygieneartikel nach Anspruch 1 zur Verwendung mit einem Baumwoll-Slip-Kleidungsstück, wobei der Artikel Mittel (6) zum lösbaren Befestigen desselben an der Innenseite des Slip-Kleidungsstückes in dessen Schrittbereich aufweist, wobei die Spannungs-Dehnungs-Eigenschaften des Artikels in einem Wesentlichen Umfang denjenigen des Schrittbereiches entsprechen.

**42.** Absorbierender Artikel nach Anspruch 1 zur Klebstoff-Befestigung an einer Unterkleidung, wobei Klebstoff auf einer Oberfläche des Artikels aufgebracht ist, wobei die Oberfläche nach außen durch eine äußere Begrenzung definiert ist, wobei der Klebstoff nur auf mindestens einen Abschnitt der Oberfläche an oder benachbart zu der äußeren Begrenzung aufgebracht ist, wobei der Rest der Oberfläche klebstofffrei ist.

**43.** Artikel nach Anspruch 42, wobei mindestens 25 % der Oberfläche klebstofffrei ist.

**44.** Artikel nach Anspruch 43, wobei mindestens 50 % der Oberfläche klebstofffrei ist.

**45.** Artikel nach einem der Ansprüche 42 bis 44, wobei der Abschnitt aus einem Streifen besteht, der um die Oberfläche benachbart zu der äußeren Begrenzung verläuft.

**46.** Artikel nach Anspruch 45, wobei der Streifen vollständig zu der äußeren Begrenzung verläuft.

**47.** Artikel nach Anspruch 45, wobei der Streifen von der äußeren Begrenzung durch einen engen klebstofffreien Bereich über dessen gesamte Länge getrennt ist.

**48.** Artikel nach Anspruch 45, wobei der Streifen von der äußeren Begrenzung durch einen engen klebstofffreien Bereich, außer über einen kleinen Abschnitt von diesem, getrennt ist.

**49.** Artikel nach Anspruch 45, wobei der Streifen vollständig zu der äußeren Begrenzung, außer über einen kleinen Abschnitt von diesem, verläuft.

**50.** Artikel nach einem der Ansprüche 42 bis 44, wobei der Klebstoff auf ein Paar der Abschnitte aufgebracht ist, wobei die Abschnitte voneinander durch einen klebstofffreien Bereich getrennt sind.

**51.** Artikel nach Anspruch 50, welcher länglich ist, und wobei das Abschnitte-Paar selbst länglich ist und seitlich entlang gegenüberliegender Ränder des Artikels verläuft, wobei der klebstofffreie Bereich ebenfalls länglich ist.

**52.** Artikel nach einem der Ansprüche 45 bis 49, welcher in Form einer Slip-Einlage vorliegt und in welchem der Streifen 4 bis 10 mm breit ist.

**53.** Artikel nach Anspruch 52, wobei der Streifen ungefähr 5 mm breit ist.

**54.** Artikel nach Anspruch 52 oder 53, ebenso abhängig von Anspruch 6 oder 7, wobei der enge klebstofffreie Bereich nicht breiter als 3 mm ist.

**55.** Artikel nach Anspruch 54, wobei der enge klebstofffreie Bereich 1 bis 2 mm breit ist.

**56.** Artikel nach Anspruch 52 oder 53, ebenso abhängig von Anspruch 45, wobei der kleine Abschnitt durch eine Vielzahl klebstofffreier Bereiche gebildet ist, die voneinander beabstandet angeordnet sind und jeweils nicht breiter als ungefähr 2 mm sind.

**57.** Artikel nach Anspruch 51, welcher in Form einer Slip-Einlage vorliegt und in welchem der längliche klebstofffreie Bereich zwischen 15 und 43 mm breit ist.

**58.** Artikel nach einem der Ansprüche 42 bis 57, welcher elastisch dehnbar ist.

**59.** Artikel nach einem der Ansprüche 42 bis 47, welcher nicht-elastisch dehnbar ist.

**60.** Artikel nach einem der Ansprüche 42 bis 57, welcher nicht-dehnbar ist.

**61.** Artikel nach einem der Ansprüche 42 bis 60, wobei der Klebstoff auf einem Styrol-Ethylen-Butylen-Styrol-Block-Copolymer basiert.

**62.** Dehnbarer absorbierender Artikel nach Anspruch 1 zur Klebstoff-Befestigung an einer Unterkleidung, wobei ein dehnbarer Klebstoff auf einer Oberfläche des Artikels aufgebracht ist, wobei die Oberfläche nach außen durch eine äußere Begrenzung definiert ist, wobei der Klebstoff auf einen Hauptabschnitt der Oberfläche aufgebracht ist und mindestens im Wesentlichen zu der äußeren Begrenzung über einen Hauptabschnitt der äußeren Begrenzung verläuft.

**63.** Artikel nach Anspruch 62, wobei der Klebstoff vollständig zu der äußeren Begrenzung über einen Hauptabschnitt der Begrenzung verläuft.

**64.** Artikel nach Anspruch 63, wobei die Oberfläche mindestens einen klebstofffreien Bereich aufweist.

**65.** Artikel nach Anspruch 64, welcher länglich ist und einen klebstofffreien Bereich an jedem Ende davon aufweist.

**66.** Artikel nach Anspruch 64, welcher eine Sanduhr-Form aufweist und einen klebstofffreien Bereich an jeder Ecke davon aufweist.

**67.** Artikel nach Anspruch 62, wobei die Oberfläche einen klebstofffreien Bereich in Form eines engen klebstofffreien Streifens aufweist, der entlang mindestens eines wesentlichen Abschnittes der äußeren Begrenzung verläuft.

**68.** Artikel nach Anspruch 67, wobei der Streifen vollständig entlang der äußeren Begrenzung verläuft.

**69.** Artikel nach einem der Ansprüche 62 bis 68, welcher in Form einer Slip-Einlage vorliegt.

**70.** Artikel nach Anspruch 65, welcher in Form einer Slip-Einlage vorliegt und in welchem jeder der klebstofffreien Bereiche eine Distanz zwischen 2 und 4 mm von dem benachbarten Ende der Slip-Einlage verläuft.

**71.** Artikel nach Anspruch 70, wobei die Distanz ungefähr 3 mm beträgt.

**72.** Artikel nach Anspruch 66, welcher in Form einer Slip-Einlage vorliegt und in welchem jeder der klebstofffreien Bereiche eine Distanz zwischen 2 und 4 mm von dem benachbarten seitlichen Rand der Slip-Einlage verläuft.

**73.** Artikel nach Anspruch 72, wobei die Distanz ungefähr 3 mm beträgt.

**74.** Artikel nach Anspruch 67 oder 68, welcher in Form einer Slip-Einlage vorliegt und in welchem der klebstofffreie Streifen eine Breite von weniger als 3 mm aufweist.

**75.** Artikel nach Anspruch 74, wobei die Breite von 1 bis 2 mm reicht.

**76.** Artikel nach einem der Ansprüche 62 bis 75, wobei der dehnbare Klebstoff auf einem Styrol-Ethylen-Butylen-Styrol-Block-Copolymer basiert.

**77.** Artikel nach einem der Ansprüche 62 bis 67, welcher elastisch dehnbar ist.

**Revendications**

**1.** Article hygiénique absorbant les fluides, multicouche, à utiliser avec une culotte vêtement, l'article ayant des moyens (6) de fixation à l'intérieur du vêtement, dans la région d'entrejambe de celui-ci, **caractérisé en ce que** l'article est flexible, et élastique dans au moins une direction, l'élasticité étant telle que, dans ladite au moins une direction, la force nécessaire pour étendre une éprouvette du matériau de 2,54 cm (1 pouce) de large jusqu'à 125 % de sa longueur d'origine n'est pas supérieure à 400 g, et l'étirement résiduel (rémanence du tampon) après que l'éprouvette a été étirée trois fois jusqu'à 125 % de sa longueur d'origine et relâchée après chaque étirement n'est pas supérieur à 15 % de sa longueur d'origine.

**2.** Article selon la revendication 1, dans lequel ladite force n'est pas supérieure à 250 g.

**3.** Article selon la revendication 2, dans lequel ladite force n'est pas supérieure à 150 g.

**4.** Article selon la revendication 3, dans lequel ladite force n'est pas supérieure à 100 g.

**5.** Article selon l'une quelconque des revendications précédentes, dans lequel ladite force est d'au moins 60 g.

**6.** Article selon l'une quelconque des revendications précédentes, dans lequel ladite rémanence du tampon n'est pas supérieure à 12 %.

**7.** Article selon la revendication 6, dans lequel ladite rémanence du tampon n'est pas supérieure à 8 %.

8.  Article selon l'une quelconque des revendications précédentes, ayant une épaisseur inférieure à 2 mm, lorsqu'elle est mesurée sous une pression de 1961 Pa (20 g/cm$^2$).

9.  Article selon la revendication 8, dans lequel l'épaisseur sous ladite pression est comprise entre 0,5 et 1,6 mm.

10. Article selon l'une quelconque des revendications précédentes, qui a une flexibilité non supérieure à 5000 mg.cm, lorsqu'elle est mesurée selon la norme ASTM D 1388-64.

11. Article selon la revendication 10, dans lequel la flexibilité n'est pas supérieure à 3000 mg.cm.

12. Article selon la revendication 10 ou 11, dans lequel la flexibilité est d'au moins 500 mg.cm.

13. Article selon l'une quelconque des revendications précédentes, dans lequel l'article est allongé et a une direction d'élasticité transversale à sa longueur.

14. Article selon l'une quelconque des revendications 1 à 12, dans lequel l'article est allongé et a une direction d'élasticité parallèle à sa longueur.

15. Article selon l'une quelconque des revendications 1 à 12, dans lequel l'article est allongé et a des directions d'élasticité transversale et parallèle à sa longueur.

16. Article selon l'une quelconque des revendications 13 à 15, dans lequel l'article a la forme d'un sablier.

17. Article selon l'une quelconque des revendications précédentes, dans lequel les couches sont de forme et de taille essentiellement identiques les unes aux autres, et sont essentiellement alignées entre elles.

18. Article selon l'une quelconque des revendications précédentes, qui comprend une couche de matériau absorbant (3) exposée à l'extérieur sur l'une de ses faces, et réunie en relation de face à face, sur sa face opposée, au moyen d'une liaison adhésive, avec une feuille de fond (5) constituée d'un matériau imperméable à l'eau.

19. Article selon l'une quelconque des revendications 1 à 17, qui comprend une couche de matériau absorbant (3) prise en sandwich entre une feuille de dessus (1) constituée d'un matériau perméable à l'eau et une feuille de fond (5) constituée d'un matériau imperméable à l'eau, le matériau absorbant (3) étant réuni à la feuille de fond (5) et à la feuille de dessus (1) par des liaisons adhésives (2).

20. Article selon la revendication 18 ou 19, dans lequel au moins le matériau absorbant (3) est élastique, et chacune des autres couches est élastique ou bien étirable mais non élastique.

21. Article selon la revendication 18, 19 ou 20, dans lequel au moins la feuille de fond (5) est élastique, et chacune des autres couches est élastique ou bien étirable mais non élastique.

22. Article selon l'une quelconque des revendications 19 à 21, dans lequel au moins la feuille de dessus (1), lorsqu'elle est prévue, est élastique, et chacune des autres couches est élastique ou bien étirable mais non élastique.

23. Article selon la revendication 22, dans lequel la feuille de dessus (1) est une structure ayant des perforations (30) qui s'étendent à travers elle, la structure comprenant :

    (a) une couche supérieure (110) destinée à être dirigée vers l'extérieur du corps absorbant, et comprenant un matériau fibreux non tissé ;
    (b) une couche intermédiaire (111) comprenant un film élastique ; et
    (c) une couche inférieure (112) destinée à être dirigée vers l'intérieur du corps absorbant, et comprenant un matériau fibreux non tissé ; les couches supérieure et inférieure (110, 112) étant reliées à la couche intermédiaire (111) essentiellement uniquement autour du périmètre des perforations.

24. Article selon l'une quelconque des revendications 18 à 23, dans lequel au moins la liaison adhésive entre le matériau absorbant et la feuille de fond (5) est élastique, et chacune des autres couches est élastique ou bien étirable mais non élastique.

**25.** Article selon l'une quelconque des revendications 19 à 24, dans lequel au moins la liaison adhésive (2) entre le matériau absorbant (3) et la feuille de dessus (1), lorsqu'elle est prévue, est élastique, et chacune des autres couches est élastique ou bien étirable mais non élastique.

**26.** Article selon l'une quelconque des revendications 18 à 25, dans lequel au moins l'un des éléments : feuille de dessus (1) (lorsqu'elle est prévue), matériau absorbant (1), feuille de fond (5), et liaison(s) adhésive(s) (2), est étirable mais non élastique.

**27.** Article selon l'une quelconque des revendications 18, 19 et 21 à 26, dans lequel le matériau absorbant (3) est étirable mais non élastique, et est choisi dans le groupe constitué des matériaux filés-lacés, des matériaux crêpés, et des matériaux fendus.

**28.** Article selon l'une quelconque des revendications 18 à 21 et 23 à 27, dans lequel la feuille de dessus (1), lorsqu'elle est prévue, est étirable mais non élastique, et est un matériau soumis à un roulage annulaire.

**29.** Article selon l'une quelconque des revendications 18 à 20 et 22 à 28, dans lequel la feuille de fond (5) est étirable mais non élastique, et est formée d'un matériau filé-lacé.

**30.** Article selon l'une quelconque des revendications 18 à 28, dans lequel la feuille de fond (5) est élastique, et est formée d'un film élastomère.

**31.** Article selon l'une quelconque des revendications 18 à 30, dans lequel la ou les liaisons adhésives (2) sont formées par une composition adhésive fusible à la chaleur, élastomère, comprenant au moins un élastomère thermoplastique et au moins une résine donnant du collant, le ou les élastomères thermoplastiques étant un copolymère styrène/butadiène/styrène (SBS) ou un mélange de styrène/butadiène/styrène avec du styrène/isoprène/styrène (SIS), dans lequel le SIS est présent en une quantité égale ou inférieure à 50 % en poids du copolymère bloc total, la composition étant **caractérisée en ce que** :

   (a) elle est capable de se lier, lorsqu'elle est appliquée à l'état fondu, à des matériaux plastiques et/ou cellulosiques, avec une force de résistance à l'arrachement à 90° non inférieure à 0,5 N/cm ;
   (b) elle a une retenue de la résistance à la traction après 50 cycles (telle que définie ici) d'au moins 40 % ;
   (c) elle a une viscosité de 120 000 cps ou moins à 180°C et sous un cisaillement appliqué de 80 s$^{-1}$.

**32.** Article selon l'une quelconque des revendications 1 à 17, dans lequel au moins l'une des couches est élastique, et chacune des autres couches est élastique ou bien étirable mais non élastique.

**33.** Article hygiénique absorbant les fluides, élastique, selon la revendication 1, à utiliser avec une culotte vêtement, l'article ayant des moyens (6) de fixation à l'intérieur du vêtement, dans la région d'entrejambe de celui-ci, l'article comprenant une pluralité de couches, réunies entre elles en une relation de face à face, au moyen d'au moins une liaison adhésive, ladite liaison adhésive ou l'une desdites liaisons adhésives étant formée par un adhésif élastique, de façon à former, au moins en partie, l'élasticité de l'article.

**34.** Article selon la revendication 33, qui comprend une couche de matériau absorbant (3) exposée à l'extérieur sur l'une de ses faces, et réunie en relation de face à face, sur sa face opposée, au moyen d'une liaison adhésive formée par un adhésif élastique, avec une feuille de fond (5) constituée d'un matériau imperméable à l'eau.

**35.** Article selon la revendication 33, qui comprend une couche de matériau absorbant (3) prise en sandwich entre une feuille de dessus (1) constituée d'un matériau perméable à l'eau et une feuille de fond (5) constituée d'un matériau imperméable à l'eau, le matériau absorbant (3) étant réuni à la feuille de fond (5) et à la feuille de dessus (1) par des liaisons adhésives, dont au moins une est formée par un adhésif élastique.

**36.** Article selon l'une quelconque des revendications 33 à 35, dans lequel l'adhésif est un adhésif fusible à la chaleur, élastomère.

**37.** Article absorbant les fluides selon la revendication 1, à utiliser avec une culotte vêtement, qui comprend une couche de matériau absorbant exposée à l'extérieur sur l'une de ses faces, et réunie en relation de face à face, sur sa face opposée, au moyen d'une liaison adhésive, avec une feuille de fond constituée d'un matériau imperméable à l'eau.

**38.** Article selon la revendication 37, ayant une épaisseur inférieure à 2 mm.

**39.** Article selon la revendication 37, ayant une épaisseur inférieure à 1 mm.

**40.** Article hygiénique absorbant les fluides selon l'une quelconque des revendications précédentes, dont les couches, et les liaisons entre elles, sont propres à le rendre perméable à la vapeur d'eau ou à d'autres gaz.

**41.** Article absorbant les fluides selon la revendication 1, à utiliser avec une culotte vêtement en coton, l'article ayant des moyens (6) pour le fixer de manière amovible à l'intérieur de la culotte vêtement, dans la région d'entrejambe de celle-ci, où les caractéristiques contrainte-déformation dudit article correspondent dans une certaine mesure à celles de ladite région d'entrejambe.

**42.** Article absorbant selon la revendication 1, à attacher de manière adhésive à un sous-vêtement, dans lequel un adhésif est appliqué sur une surface de l'article, ladite surface étant définie extérieurement par un périmètre, l'adhésif étant appliqué uniquement sur au moins une partie de ladite surface, au niveau ou en contiguïté dudit périmètre, le restant de la surface étant exempt d'adhésif.

**43.** Article selon la revendication 42, dans lequel au moins 25 % de ladite surface sont exempts d'adhésif.

**44.** Article selon la revendication 43, dans lequel au moins 50 % de ladite surface sont exempts d'adhésif.

**45.** Article selon l'une quelconque des revendications 42 à 44, dans lequel ladite partie consiste en une bande s'étendant autour de ladite surface contiguë audit périmètre.

**46.** Article selon la revendication 45, dans lequel ladite bande s'étend complètement sur ledit périmètre.

**47.** Article selon la revendication 45, dans lequel ladite bande est séparée dudit périmètre par une étroite région exempte d'adhésif, sur toute la longueur de celui-ci.

**48.** Article selon la revendication 45, dans lequel ladite bande est séparée dudit périmètre par une étroite région exempte d'adhésif, sauf sur une petite partie de celui-ci.

**49.** Article selon la revendication 45, dans lequel ladite bande s'étend complètement sur le périmètre, sauf sur une petite partie de celui-ci.

**50.** Article selon l'une quelconque des revendications 42 à 44, dans lequel l'adhésif est appliqué sur deux desdites parties, lesdites parties étant séparées l'une de l'autre par une région exempte d'adhésif.

**51.** Article selon la revendication 50, qui est allongé, et dans lequel lesdites deux parties sont elles-mêmes allongées, et s'étendent le long de bords de l'article opposés dans la direction latérale, la région exempte d'adhésif étant également allongée.

**52.** Article selon l'une quelconque des revendications 45 à 49, qui se présente sous la forme d'un protège-slip, et dans lequel ladite bande fait 4 à 10 mm de large.

**53.** Article selon la revendication 52, dans lequel ladite bande fait environ 5 mm de large.

**54.** Article selon la revendication 52 ou 53, lorsqu'elles dépendent de la revendication 6 ou 7, dans lequel ladite étroite région exempte d'adhésif ne fait pas plus de 3 mm de large.

**55.** Article selon la revendication 54, dans lequel ladite étroite bande exempte d'adhésif fait entre 1 et 2 mm de large.

**56.** Article selon la revendication 52 ou 53, lorsqu'elles dépendent de la revendication 45, dans lequel ladite petite partie est constituée d'une pluralité de régions exemptes d'adhésif espacées les unes des autres et ne faisant chacune pas plus d'environ 2 mm de large.

**57.** Article selon la revendication 51, qui se présente sous la forme d'un protège-slip, et dans lequel la région exempte d'adhésif allongée fait entre 15 et 43 mm de large.

**58.** Article selon l'une quelconque des revendications 42 à 57, qui est étirable de manière élastique.

**59.** Article selon l'une quelconque des revendications 42 à 47, qui est étirable de manière non élastique.

**60.** Article selon l'une quelconque des revendications 42 à 57, qui est non étirable.

**61.** Article selon l'une quelconque des revendications 42 à 60, dans lequel ledit adhésif est à base d'un copolymère bloc styrène/éthylène/butylène/styrène.

**62.** Article absorbant étirable selon la revendication 1, à attacher de manière adhésive à un sous-vêtement, dans lequel un adhésif étirable est appliqué sur une surface de l'article, ladite surface étant définie extérieurement par un périmètre, l'adhésif étant appliqué sur une grande partie de ladite surface, et s'étendant au moins essentiellement sur ledit périmètre, sur une grande partie dudit périmètre.

**63.** Article selon la revendication 62, dans lequel l'adhésif s'étend complètement sur ledit périmètre, sur une grande partie dudit périmètre.

**64.** Article selon la revendication 63, dans lequel ladite surface a au moins une région exempte d'adhésif.

**65.** Article selon la revendication 64, qui est allongé et qui a une région exempte d'adhésif à chacune de ses extrémités.

**66.** Article selon la revendication 64, qui a la forme d'un sablier, et qui a une région exempte d'adhésif à chacun de ses coins.

**67.** Article selon la revendication 62, dans lequel ladite surface a une région exempte d'adhésif se présentant sous la forme d'une étroite bande exempte d'adhésif, s'étendant le long d'au moins une partie importante dudit périmètre.

**68.** Article selon la revendication 67, dans lequel ladite bande s'étend complètement le long dudit périmètre.

**69.** Article selon l'une quelconque des revendications 62 à 68, qui se présente sous la forme d'un protège-slip.

**70.** Article selon la revendication 65, qui se présente sous la forme d'un protège-slip, et dans lequel chacune desdites régions exemptes d'adhésif s'étend sur une distance de 2 à 4 mm à partir de l'extrémité contiguë du protège-slip.

**71.** Article selon la revendication 70, dans lequel ladite distance est égale à environ 3 mm.

**72.** Article selon la revendication 66, qui se présente sous la forme d'un protège-slip, et dans lequel chacune desdites régions exemptes d'adhésif s'étend sur une distance de 2 à 4 mm à partir du bord latéral contigu du protège-slip.

**73.** Article selon la revendication 72, dans lequel ladite distance est égale à environ 3 mm.

**74.** Article selon la revendication 67 ou 68, qui se présente sous la forme d'un protège-slip, et dans lequel ladite région exempte d'adhésif a une largeur inférieure à 3 mm.

**75.** Article selon la revendication 74, dans lequel ladite largeur est comprise entre 1 et 2 mm.

**76.** Article selon l'une quelconque des revendications 62 à 75, dans lequel ledit adhésif étirable est à base d'un copolymère bloc styrène/éthylène/butylène/styrène.

**77.** Article selon l'une quelconque des revendications 62 à 76, qui est étirable de manière élastique.

Fig.1.

Fig.2a.

Fig.2b.

PANTY 100 % cotton

Fig.3.

EP 0 784 459 B1

product # 1

Fig.4.

% elongation

force (N/inch)

product # 2

Fig.5.

EP 0 784 459 B1

product # 4

% elongation

Fig.6.

force(N/inch)

Fig.7.

EP 0 784 459 B1

product # 8

Fig.8.

% elongation

force (N/inch)

Fig.9.

EP 0 784 459 B1

Fig.10.

Fig.11.

EP 0 784 459 B1

Fig.12.

Fig.13.

EP 0 784 459 B1

## Fig.14.

## Fig.15.

Fig.16.

Fig.20.

Fig.19.

Fig.18.

Fig.17.

Fig.23.

Fig.22.

Fig.21.

Fig.24.

Fig.25.

Fig.26.

Fig.27.